# EUROPEAN PATENT SPECIFICATION

(11) **EP 4 272 745 B1**
(45) Date of publication and mention of the grant of the patent: **20.11.2024**
(21) Application number: 23170459.4
(22) Date of filing: 27.04.2023
(51) Int. Cl.: A61K 31/704, A61K 31/724, A61K 33/00, A61K 33/24, A61P 31/00

(54) **PHARMACEUTICAL COMPOSITION FOR RESTORING PHYSIOLOGICAL PROCESSES AND CELLS OF ORGANISM**
PHARMAZEUTISCHE ZUSAMMENSETZUNG ZUR WIEDERHERSTELLUNG PHYSIOLOGISCHER PROZESSE UND ZELLEN EINES ORGANISMUS
COMPOSITION PHARMACEUTIQUE POUR RESTAURER DES PROCESSUS PHYSIOLOGIQUES ET DES CELLULES D'ORGANISME

(30) Priority: 05.05.2022 WO PCT/IB2022/054147
(43) Date of publication of application: 08.11.2023
(73) Proprietor: Kurkayev, Abdula, 1097 Budapest (HU)
(72) Inventor: KURKAYEV, Abdula, 1097 Budapest (HU); SZENTE, Lajos, 1118 Budapest (HU); PUSKAS, Istvan, 1188 Budapest (HU)
(74) Representative: Botti & Ferrari S.p.A.

(56) References cited:
- WO-A1-2011/151667
- CN-A- 104 353 105
- CN-A- 106 797 954
- ZHANG HUI ET AL: "Study on Photocatalytic Antibacterial and Sustained-Release Properties of Cellulose/TiO 2 / [beta] -CD Composite Hydrogel", vol. 2019, 24 July 2019 (2019-07-24), US, pages 1 - 12, XP093001301, ISSN: 1687-4110, Retrieved from the Internet <URL:http://downloads.hindawi.com/journals/jnm/2019/2326042.xml> DOI: 10.1155/2019/2326042
- SELVAM S ET AL: "Antibacterial effect of novel synthesized sulfated -cyclodextrin crosslinked cotton fabric and its improved antibacterial activities with ZnO, TiOand Ag nanoparticles coating", INTERNATIONAL JOURNAL OF PHARMACEUTICS, ELSEVIER, NL, vol. 434, no. 1, 25 April 2012 (2012-04-25), pages 366 - 374, XP028428074, ISSN: 0378-5173, [retrieved on 20120503], DOI: 10.1016/J.IJPHARM.2012.04.069
- SHI JINJIN ET AL: "Reactive Oxygen Species-Manipulated Drug Release from a Smart Envelope-Type Mesoporous Titanium Nanovehicle for Tumor Sonodynamic-Chemotherapy", APPLIED MATERIALS & INTERFACES, vol. 7, no. 51, 21 December 2015 (2015-12-21), US, pages 28554 - 28565, XP093001388, ISSN: 1944-8244, DOI: 10.1021/acsami.5b09937

## Description

### FIELD OF THE INVENTION

The present invention relates to pharmaceutical compositions and methods for making and using the compositions. Moreover, the present invention concerns compositions that exhibit improvement for restoring the physiological processes of cells and the body via synergistic interaction between organic and inorganic components, thus are suitable for - amongst other beneficial effects - decreasing the incidence of microbial, bacterial, fungal and viral infections via interaction with cell membrane components and also generation of reactive oxygen species. Improvement for restoring the physiological processes of cells and the body can be controlled both in a curative and preventive manner by local or systemic administration of parent and chemically modified cyclic oligosaccharides (called cyclodextrins) together with nano-sized metal or metalloid oxide particles (such as titanium-dioxide and silicon-dioxide) dispersed in water.

### DETAILED DESCRIPTION OF THE INVENTION

Improvement for restoring the physiological processes of cells and the body of certain nanoparticles alone and by UV-visible light, has been thoroughly studied, and is well known.

Cyclodextrins are enzyme-modified starch derivatives prepared from pre-hydrolyzed starch by the action of CTG-ase (cyclodextrin-glycosyl transferase) enzyme. (Szejtli, J.:Cyclodextrin Technology, 1988. Kluwer Academic Publ. Co. Dordrecht, the Netherlands) Cyclodextrins comprise glucose units arranged to a torus, by alpha-1,4-glycosidic linkages.

The most commonly used types of cyclodextrins are 6-membered alpha-cyclodextrin, 7-membered beta-cyclodextrin, and 8-membered gamma-cyclodextrins, respectively. Also, cyclodextrin derivatives such as randomly methylated beta-cyclodextrin (abbr.: RAMEB), 2-hydroxypropyl-beta-cyclodextrin (abbr.: HPBCD), sulfobutylated beta-cyclodextrin (abbr.: SBECD), 6A,6B,6C,6D,6E,7F,6G,6H-Octakis-S-(2-carboxyethyl)-6A,6B,6C,6D,6E,6F,6G,6H-octathio-gamma-cyclodextrin (INN/common name: sugammadex) or 2-hydroxypropyl-gamma-cyclodextrin (abbr.: HPGCD) are widely used in healthcare products. Cyclodextrins have been disclosed as being useful for decreasing viral infections and against cancer alone and in combinations with antiviral/antineoplastic, cytostatic pharmaceutical actives.

Cyclodextrins have also been used to form inclusion complexes with antimicrobial pharmaceutical active drugs as a means of ensuring the improved water solubility, enhanced delivery of the active compounds to a patient in need thereof as described, for example by the following examples:
Cyclodextrin complexes with ltraconazol and combinations are described as improved aqueous solubility and better bioavailable forms of the antifungal drug. Clin. Pharmacol. (1998), 38(7), 593-602; Antimicrob. Agents Chemother. (1997), 41(11), 2554-2558
WO 2000023454 invention discloses ribavirin/cyclodextrin combinations. A method for treating a patient having chronic hepatitis C infection to eradicate detectable HCV-RNA involving a combination therapy using a therapeutically effective amount of ribavirin derivatives in suitable combinations with hydroxypropyl-beta-cyclodextrins to improve the oral bioavailability of drugs in vivo.

US Patent 687599 discloses the preparation and use of azidothimydine-cyclodextrin combinations as potent antiviral agent. The said antiviral composition comprise an effective amount of alpha-cyclodextrin-sulfate a pharmacologically acceptable carrier. The antiviral composition may also contain an additional other known antiviral agent, e.g. AZT, glucocorticoid. Infection with retroviruses (particularly with HIV) can be treated by administering to a patient an effective amount of the said composition alone or in combination with other known antiviral agents. The preparation (by a modified known procedure), the viral inhibition effectiveness and toxicity of alpha-cyclodextrin-sulfate, as well as the comparison and enhancement of the viral inhibition effectiveness of alpha-cyclodextrin sulfate with other antiviral agents are disclosed.

Cyclodextrin complexed form of gancyclovir has been used to provide enhanced bioavailability of the antiviral agent. (Bioorganic & Medicinal Chemistry; 9 (2) 275-282, 2001.)

Use of 2-hydroxypropylated beta-cyclodextrin HPBCD itself to control viral infections has already been described in a number of previous publications.
(see for instance papers in Journal of Virology, 77(15), 8237-8248 2003; AIDS Research and Human Retroviruses (2001), 17(11), 1009-1019.)

Vaginal transmission of cell-associated HIV-1 in the mouse is blocked by beta-cyclodextrin, a topical, membrane-modifying agent. Topical application of beta-cyclodextrin, a cholesterol-sequestering agent that interferes with cell migration and budding of virus from lipid rafts, blocks transmission of cell-associated HIV-1.(see Journal of Clinical Investigation, 109 (2), 205-211, 2002.)

It has also been published that cholesterol in HIV virions is strictly required for fusion and viral infectivity. (AIDS Research and Human Retroviruses, 19 (8), 675-687 2003.) These observations in combination with those of past studies indicate beta-cyclodextrin to be an excellent candidate for use as a chemical barrier for AIDS prophylaxis. The cholesterol-binding methyl-beta-cyclodextrin was found to reduce SARS-Cov-2 viral infection in cell test in vitro, and positive correlation was found between recipient cells cholesterol status and the virus infectivity (Glende, M et al : Virology 2008 Nov 25; 381(2): 215-221.).

Among the suitable organic components of the antimicrobial compositions according to the present invention, are natural and (ionic and non-ionic) synthetic mono-, di-, and oligosaccharides. Such compounds are for instance pentosan-polysulfate, dextrose-sulfate, heparinoids, natural heparin, e.t.c. known to have potent antimicrobial antiviral effects. (Baba, M et al Antimicrobial Agents and Chemotherapy Nov. 1998. 1742-1745., Ito et al Antiviral Res. 7. 361-367. 1987., Mitsuya et al Science 240, 646-649. 1988.; Nahmias etr al J. Bacteriol. 87. 1060-1066. 1964.) Sulfated alpha- and beta-cyclodextrins in combination with known antiviral drug actives were found to have synergistic viricide effect. (Antiviral Chem. Chemother. 4. 1. pp 65-66. 1993. and Antiviral Chem. 1 . 1. 41-46. 1990.

Other types of sulfated macromolecules also exhibit antiviral effect. Three of these products PRO2000, Carraguard and cellulose-sulfate are anionic polymers and inhibit HIV-1 infection by preventing virus-cell fusion predominantly through charged based interactions. In addition, Viva Gel (SPL7013, a sulphated dendrimer) thought to work by a similar mechanism has entered in early phase I safety trials. Another antimicrobial product in phase III trials is a buffer gel (Buffergel) containing polyanionic carbopol. These agents inhibit both HIV and HSV in cell culture and animal models.

Besides linear polysaccharides cyclic oligosaccharides including cycloaltrins, cyclofructins, cyclodextrins, cyclomannins etc. in their natural forms and in particular when bearing anionic substituents represent a class of substances that are known to interact with certain compounds occurring in cell surfaces and thus providing remarkable antimicrobial effects. In particular, sulfated-,sulfoalkylated and carboxyalkylated negatively charged types of cyclic oligosaccharides are known to exert such a microbial potency.

WO 2003/080079 discloses the use of sulfobutyl ether cyclodextrin as a preservative. The method includes the step of including a derivatized cyclodextrin in a formulation capable of sustaining microbial growth. One embodiment of the formulation employs a sulfoalkyl ether cyclodextrin as a preservative and optionally as a solubilizing and complexing agent. A suitable cyclodextrin is the Dexolve brand cyclodextrin (sulfobutyl ether beta-cyclodextrin). Whether or not the formulation includes a conventional preservative, the formulation will remain preserved for at least a minimum predetermined period. Specific embodiments of the invention include a carrier, a derivatized cyclodextrin and optionally one or more active agents, one or more water activity-reducing agents, and/or one or more complexation-enhancing agents. The derivatized cyclodextrin reduces the water activity of the formulation. A liquid formulation can be lyophilized or otherwise dried to yield a solid formulation that is optionally reconstitutable.

Combinations of organic oligo- and polymens with inorganic nanoparticles Jpn. Kokai Tokkyo Koho JP 2009209075 A 17 Sep 2009 Describes titanium dioxide-apatite complex particles and cyclodextrins claimed as antibacterials for oral hygiene to remove virus and inflammation in the oral cavity and pharynx. The title antibacterials can be used as oral sustained-release preparations, buccal agents, and troches. The agent for oral hygiene aiming at the removal of the bacteria, which replace the hygienic mask or contain the virus in the oral cavity and the pharynx with use of the hygienic mask is provided concerning the agent for oral hygiene for making the prevention and therapy of the inflammatory disease in the removal of the bacteria containing the virus in the oral cavity and the pharynx, the oral cavity, and the pharynx. It is considered as the agent for oral hygiene containing titanium dioxide particles and cyclodextrin. Titanium dioxide particles suitably form complex particles with the apatite. By these actions, the inflammation in the removal of the bacteria containing the virus in the oral cavity and the pharynx, the oral cavity, and the pharynx is claimed.

Antiviral Research, 81(3), 261-266 2009 describes the use of Ribavirin (RBV) a water-soluble synthetic nucleoside with broad spectrum antiviral properties. However, it is ineffective against major viral encephalitis because of a failure to cross the blood-brain barrier (BBB). The antiviral activity of the complexed drug the ribavirin/alpha-cyclodextrin complex was previously demonstrated to be stronger than free ribavirin, in an in vivo model of measles virus (MV) encephalitis in mice. The role of cyclodextrin on ribavirin uptake into the brain needs to be defined. Ribavirin specific extraction from brain tissue was developed, based on a solid phase extraction. It was quantified by high performance liquid chromatography at different time points after i.p. injection of single or multiple doses of free ribavirin or of the complex ribavirin/alpha-cyclodextrin. Whatever the tested dose (40 or 100 mg/kg), the amount of ribavirin in the brain was significantly higher (p < 0.001) when the drug was injected as a complex with alpha-cyclodextrin, in healthy or measles virus-infected mice.

US 20080242636 A1 2 Oct 2008 :This invention relates to method of reducing human immunodeficiency virus (HIV) infective events by applying cyclodextrin sulfate-71 (terygyn) and a suitable excipient formulated for topical administration to the vaginal, anal, urethral or penile glans mucosa of an individual prior to or subsequent to infection by human immunodeficiency virus.

PCT Int. Appl. WO 2008063634 A1 29 May 2008: The present invention relates to pharmaceutical formulations of benzodiazepine compounds, which are active against respiratory syncytial virus (RSV), suitable for parenteral administration for treatment of a RSV infection in pediatric patients. Thus, 6 mg/mL (S)-1-(2-fluorophenyl)-3-(2-oxo-5-phenyl-2,3-dihydro-1H-benzo[e][1,4]diazepin-3-yl)urea (free base equiv.) was dissolved in 40% hydroxypropyl beta-cyclodextrin, with addition of 15 mM phosphate buffer, at pH 7. The lyophilized cake of this solution was reconstituted with 3.8 mL of 5% dextrose solution to obtain 4.4 mL of 3 mg/mL (S)-1-(2-fluorophenyl)-3-(2- oxo-5-phenyl-2,3-dihydro-1H-benzo[e][1,4]diazepin-3-yl)urea in 20% HPBCD.

Bencini M et al in Journal of Controlled Release, 126(1), 17-25 2008 described preparation and use of poly(amidoamine) (PAA) copolymer with beta-cyclodextrin, by reacting 6-deoxy-6-amino-beta-cyclodextrin and 2-methylpiperazine to2,2-bis(acrylamido)acetic acid. This beta-CD/PAA copolymer bears beta-cyclodextrin units along the macromolecular chain, is water-soluble and non-cytotoxic. The complexing capacity of beta-cyclodextrin based copolymer was determined using an antiviral drug, Acyclovir, as a model of poorly water-soluble drug. The antiviral activity of Acyclovir beta-CD/PAA polymer complex was evaluated against herpes simplex virus type I in cell cultures. The Acyclovir beta-CD/PAA complex exhibited a higher antiviral activity than the free drug.

PCT Int. Appl. WO 2004096121 A2 11 Nov 2004 This invention relates to cholesterol-sequestering agents and methods of using cholesterol-sequestering agents to prevent viral infection. The compounds of the invention can be used to decontaminate skin and environmental surfaces that are contacted with microorganisms such as envelope viruses.

PCT Int. Appl., WO 2000012137 A1 20000309 WO 1999-US20060 relates to compositions including a liquid medium, a cyclodextrin component and a preservative component, which has a reduced tendency to being complexed with the cyclodextrin component. In one embodiment, the preservative component is a chlorite component. Active drug components are included in the compositions. In the composition NaCl, KCI, CaCl2.2H2O, MgCl2.6H2O, Carboxymethyl-cellulose-sodium salt, boric acid, sodium borate decahydrate, brimodine tartrate, sulfobutyl ether beta-cyclodextrin and water, stabilized ClO2 50 ppm. The presence of a cyclodextrin component does not have any detrimental effect on the preservative efficacy of stabilized chlorine dioxide. The stabilized chlorine dioxide remains free and effective as a preservative rather than being complexed by the cyclodextrin component. The composition is ophthalmically acceptable. WO2011151667 discloses an antiviral composition used as e.g. personal care or pharmaceutical products comprises titanium dioxide, silicon dioxide or barium sulfate nanoparticles of specific size, (2-hydroxy)propyl beta-cyclodextrin. CN104353105 discloses a medical nano antibacterial gel comprises agarose, gamma-cyclodextrin, polyethylene glycol, boric acid, nano titanium dioxide, nano zirconium dioxide, and water. ZHANG HUI ET AL. in JOURNAL OF NANOMATERIALS, vol. 2019 24 July 2019 (2019-07-24), pages 1-12, discloses a cellulose/TiO2/β-CD hydrogel with high photocatalytic antibacterial activity and sustained release of drug. SELVAM S ET AL. in INTERNATIONAL JOURNAL OF PHARMACEUTICS, ELSEVIER, NL, vol. 434, no. 1, 25 April 2012 (2012-04-25), pages 366-374 discloses the antibacterial effect of synthesized sulfated β-cyclodextrin crosslinked cotton fabric and its improved antibacterial activities in the presence of ZnO, TiO2 and Ag nanoparticles.

The object of present invention concerns the preparation and utilization of different nanosized metal- oxide dispersions in a combination with cyclodextrins and cyclodextrins derivatives in aqueous systems to prevent and control microbial infections.

US Patent 6835717 entitled β-cyclodextrin compositions, and use to prevent transmission of sexually transmitted diseases, discloses the use of beta-cyclodextrins as chemical barriers to prevent and treat viral infections. The invention is based on the cholesterol status alteration of the virus envelope, via a reversible non-covalent inclusion complex formation between the used cyclodextrin and the surface lipids. The application of beta-cyclodextrin and its derivatives was found to effectively deplete cholesterol from the virus surface on the other hand also from the cell membrane of locally treated surfaces.

Because of their physical-chemical characteristics nanoparticles become highly useful in different fields: treatment of different diseases, for example, infectious, bacterial or viral infections, cancer therapy, wounds healing, anemia treatment etc.

From the technical field the European patent No. 016541 WO 2007/118884 2007.10.25 (PCT PCT/EP2007/053761) is known(Compositions of magnetic nanoparticles and their use).

The disclosure relates to use of biocompatible nanoparticle or nanoparticles aggregate in combination with external non-oscillating magnetic field, where said nanoparticle includes: a) a core containing magnetic material; b) biocompatible cover, surrounding the core; and, optionally, c) a marking agent, where the external diameter of the cover is less than about 100 nm, for preparation of composition, where said composition does not contain any other means for targeting on the cell. The present invention also relates to obtained compositions and their use in the health care area, for cancer treatment or in diagnostics (for example, visualization), for monitoring of the tumor development.

Ferromagnetic material is chosen from the group consisting of iron, nickel, cobalt, gadolinium, samarium, neodymium, boron, aluminium and any mixture of them. Material of ferromagnetic core is in form of oxide, hydroxide or metal.

Compositions may exist in solid or liquid form (suspended nanoparticles), for example, in the form of paste or aerosol.

### DISCLOSURE OF THE INVENTION

The invention is destined for creation of pharmaceutical compositions, ensuring restoring the physiological processes of cells and all the organism, as a whole, at the expense of generation of reactive species of oxygen (ROS) in an organism.

Pharmaceutical composition on the base of which the medical agent is claimed is characterized by the fact that it ensures therapeutically significant effect for restoration of the physiological processes and cells of an organism.

Pharmaceutical composition for restoring the physiological processes and the cells of an organism is characterized by presence of cyclic maltooligosaccharides (cyclodextrins) chosen from the group of cyclomaltooligosaccharides, namely, alpha-cyclodextrins, beta-cyclodextrins, gamma-cyclodextrins, randomly methylated beta-cyclodextrins, 2-hydroxypropyl-beta-cyclodextrins, sulfobutylated beta-cyclodextrins, 2-hydroxypropyl-gamma-cyclodextrins or their appropriate mixtures and sugammadex (6A,6B,6C,6D,6E,7F,6G,6H-octakis-S-(2-carboxyethyl)-6A,6B,6C,6D,6E,6F,6G,6H-octathio-gamma-cyclodextrin), used in amount 0.001-99.9 mass% in combination with aqueous solution of stable suspension of heterocrystals of titanium dioxide or silicon dioxide particles with size no less than 450 nm and content of titanium dioxide or silicon dioxide in amount of 0.0001 - 5% mass, having on the surface of crystals and particles in the energy centers electronically-excited triplet oxygen ³O₂

Stable aqueous suspension according to the present invention is chosen in such a way that combined use in presence of components of cyclodextrin preferably have decreased toxicity, more preferably, in essence, non-toxicity at administration of compositions to a human or an animal.

The method of obtaining stable suspension of heterocrystals of titanium dioxide or silicon dioxide particles with size less than 450 nm having on the surface of crystals and particles in the energy centers electronically-excited triplet oxygen ³O₂ is in detail described in application PCT / IB 2022/054063, filed 03/05/2022 KURKAYEV, A **"Method of obtaining stable suspensions of heterocrystals of titanium dioxide and particles of silicon dioxide and stable suspension obtained by this method for initiation of active form of oxygen in human body at use in medical forms".**

The pharmaceutical composition additionally comprises active pharmaceutical ingredients (API) and pharmaceutically acceptable additives (PAA).

Used at present time active pharmaceutical ingredients and pharmaceutically acceptable additives are preferably chosen in the way to obtain benefit from presence of cyclodextrin components.

Usually active ingredients and pharmaceutically permitted additives have increased solubility in water, due to presence of cyclodextrin components.

Pharmaceutical composition is characterized by the fact that in aqueous solution of suspension heterocrystals of titanium dioxide have the following distribution: up to 1 nm is 0.3 vol%, up to 20 nm is 5-40 vol%, particles up to 80 nm are 10-80 vol%, particles up to 150 nm are 5-30 vol%, particles up to 250 nm are 5-20 vol%, particles with a size of more than 250 nm are not more than 10 vol%, and the distribution of finely dispersed particles of silicon dioxide with a size of 40-80 nm is 10-80 %, particles with a size of 80-150 nm are 10-80 %, particles with a size of 150- 250 nm is less than 30 %, particles larger than 250 nm - no more than 15%.

Data obtaining stable suspension of heterocrystal of titanium dioxide or particles of silicon dioxide with size less than 450 nm, in the analysis with the photon correlation spectroscopy instrument Malvern Zetasizer Nano ZS

Presence on the crystal surface of oxygen O2 in the ruptures of the developed surface of crystals lattices of a great number of ionic groups of ligands ensures formation of excitonic structures, ionic bonds and zones of local energy overheating, i.e. the quantum dots, where O2 is in metastable electronically-excited triplet condition (2T3+) with unique characteristics of conversion into biological activity - singlet condition (S-1-3).

Illustration of pharmacological study of measuring quantity of electronically-excited triplet oxygen (2T3+), on particles surface. Executed as follows. Researches executed on a rabbit, male with 0.75 kg weight (without injuries).

Through the needle 5 ml of suspension was injected, where 5 ml is injection water and 3 mg TiO2, at the entry inside the needle opening there was a light guide for feeding laser photons and supplying injection suspension simultaneously.

The photonic-thermic procedure was executed with the laser IR 960 nm with 3 Wt power, dose 20 J regulating synchronously.

In the local treated part of the examined rabbit, with a help of an additional needle the sensor for measuring O₂ was installed, and the analyser of automatic chemiluminiscent gas analyser of singlet oxygen ³O₂ (the model FOMS - 200 OXYGEN, made in FRG).

Presence of conversion of triplet oxygen with presence in oxygen structure n metastable electronically-excited condition into singlet condition (S⁻¹⁻³) was fixed.

The surface of heterocrystals of titanium dioxide and silicon dioxide particles has sorption ability, that is an important factor for use in the medical forms.

Doxorubicin is a known cytostatic drug applied via intravenous route. Those skilled in the art have a concept that doxorubicin may not be applied intramuscularly due to its necrotic effect. We have surprisingly found that a particulate composition containing doxorubicin (in an amount of 0.0001 to 0.001 mg/mL) besides the nanoparticles and cyclodextrin according to present invention is suitable for intramuscular injection whereas the therapeutic function of the drug is manifested, without unwanted necrotic side-effect.

Said pharmaceutical compositions may be used intravenously, intramuscularly, orally, nasally, vaginally, rectally, ENT administration or topically and in the form of ointments, creams, lotions, solutions, powders, patches and aerosols

Pharmaceutical composition is used, in particular, in the form of medical articles of topical use in the form of hydrocolloidal medical plasters, impregnation for tampons, absorbent pads, diapers, tapes, bandages etc., having antimicrobial and antiseptic influence, anti-inflammatory action, immunomodulatory and adsorbing action, depending on form and method of their use.

Mechanism of action in composition of a medical agent or gel is that in interaction of electronically-excited O₂ on the surface of crystals or particles with membrane cellular enzyme complex NADFH ROS is formed.

Pharmaceutical composition on the base of which the medical agent is claimed is characterized by the fact that it ensures therapeutically significant effect for restoration of the physiological processes and cells of an organism.

Present invention directs to novel pharmaceutical compositions for restoring the physiological processes of cells and the body by combining cyclodextrins selected from the group of alpha-cyclodextrin, beta-cyclodextrin, gamma-cyclodextrin, randomly methylated beta-cyclodextrin, 2-hydroxypropyl-beta-cyclodextrin, sulfobutylated beta-cyclodextrin, sugammadex or 2-hydroxypropyl-gamma-cyclodextrin. Cyclodextrins (CDs) are a class of highly water-soluble and biocompatible cyclic oligosaccharides. Cyclodextrins tend to bind other molecules in their quasi-cylindrical interiors. This inclusion (and release) behaviour leads to applications in medicine. The compound is of wide interest because it exhibits host-guest properties, forming inclusion compounds. Cyclodextrins differ by size, for example, alpha-cyclodextrin composed of 6 glucose subunits, beta-cyclodextrin: 7 glucose subunits, gamma-cyclodextrin: 8 glucose subunits. Methyl-β-cyclodextrin (Methyl-beta-cyclodextrin) is a cyclic heptasaccharide used to deliver hydrophobic drugs based on its property of solubilizing non-polar substances. Methyl-β-cyclodextrin is also extensively used as a cholesterol-depleting reagent, 2-Hydroxypropyl-β-cyclodextrin is a cyclic oligosaccharide that is widely used as an enabling excipient in pharmaceutical formulations, but also as a cholesterol modifier. Sulfobutyl ether beta-cyclodextrin is a highly water-soluble anionic derivative of cyclodextrin, which ensures solubility and stability of API and their properties. Sugammadex is a modified gamma-cyclodextrin, with a lipophilic core and a hydrophilic periphery. Sugammadex is used to reverse neuromuscular blockade after administration of the aminosteroid non-depolarizing neuromuscular-blocking agents such as vecuronium or rocuronium.

Representative examples of additives include API and pharmaceutically permitted additives taken in amount 0.0001 - 99.9% mass, such as Lenalidomide, nivolumab, Imbruvica, cytostatic agents: anthracyclines (doxorubicin, epirubicin, pegylated liposomal doxorubicin), Taxanes (paclitaxel, docetaxel, albumin nano-particle bound paclitaxel), 5-fluorouracil (continuous infusion 5-FU, capecitabine), Vinca alkaloids (vinorelbine, vinblastine), Gemcitabine, Platinum salts (cisplatin, carboplatin), cyclophosphamide, Etoposide and combinations of one or more of the above such as Cyclophosphamide/anthracycline+/-5-fluorouracil regimens (such as doxorubicin/cyclophosphamide (AC), epirubicin/cyclophosphamide, (EC) cyclophosphamide/epirubicin/5-fluorouracil (CEF), cyclophosphamide/doxorubicin/5-fluorouracil (CAF), 5-fluorouracil / epirubicin / cyclophosphamide (FEC), cyclophosphamide/metothrexate/5-fluorouracil (CMF), anthracyclines/taxanes (such as doxorubicin/paclitaxel or doxorubicin / docetaxel), Docetaxel / capecitabine, Gemcitabine/paclitaxel, Taxane/platinum regimens (such as paclitaxel / carboplatin or docetaxel/carboplatin).

Representative examples of antiviral additives include: Abacavir, Acyclovir (Aciclovir), Adefovir, Amantadine, Ampligen, Amprenavir (Agenerase), Umifenovir (Arbidol), Atazanavir, Atripla, Baloxavir marboxil (Xofluza), Biktarvy, Boceprevir, Bulevirtide, Cidofovir, Cobicistat (Tybost), Combivir, Daclatasvir (Daklinza), Darunavir, Delavirdine, Descovy, Didanosine, Docosanol, Dolutegravir, Doravirine (Pifeltro), Edoxudine, Efavirenz, Elvitegravir, Emtricitabine, Enfuvirtide, Ensitrelvir, Entecavir, Etravirine (Intelence), Famciclovir, Fomivirsen, Fosamprenavir, Foscarnet, Ganciclovir (Cytovene), Ibacitabine, Ibalizumab (Trogarzo), Idoxuridine, Imiquimod, Imunovir, Indinavir, Lamivudine, Letermovir (Prevymis), Lopinavir, Loviride, Maraviroc, Methisazone, Moroxydine, Nelfinavir, Nevirapine, Nexavir formerly (Kutapressin), Nitazoxanide, Norvir, Oseltamivir (Tamiflu), Penciclovir, Peramivir, Penciclovir, Peramivir (Rapivab), Pleconaril, Podophyllotoxin, Raltegravir, Remdesivir, Ribavirin, Rilpivirine (Edurant), Rilpivirine, Rimantadine, Ritonavir, Saquinavir, Simeprevir (Olysio), Sofosbuvir, Stavudine, Taribavirin (Viramidine), Telaprevir, Telbivudine (Tyzeka), Tenofovir alafenamide, Tenofovir disoproxil, Tipranavir, Trifluridine, Trizivir, Tromantadine, Truvada, Umifenovir, Valaciclovir (Valtrex), Valganciclovir (Valcyte), Vicriviroc, Vidarabine, Zalcitabine, Zanamivir (Relenza), Zidovudine.

Representative examples of additives include excipients, fillers, soloubilizers, co-solvents, surfactants, lubricants, gelling agents, colorants, sweeteners, taste modifiers, etc, such as cetyl pyridinium chloride, gelatin, casein, lecithin (phosphatides), dextran, glycerol, gum acacia, cholesterol, tragacanth, stearic acid, calcium stearate, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene sorbitan fatty acid esters (e.g., the commercially available Tweens^{®} such as e.g., Tween 20^{®} and Tween 80^{®} (ICI Specialty Chemicals)); polyethylene glycols (e.g., Carbowaxs 3350^{®} and 1450^{®}, and Carbopol 934^{®} (Union Carbide)), dodecyl trimethyl ammonium bromide, polyoxyethylene stearates, colloidal silicon dioxide, phosphates, sodium dodecylsulfate, carboxymethylcellulose calcium, hydroxypropyl cellulose (HPC, HPC-SL, and HPC-L), hydroxypropyl methylcellulose (HPMC), carboxymethylcellulose sodium, methylcellulose, hydroxyethylcellulose, hydroxypropylcellulose, hydroxypropylmethyl-cellulose phthalate, noncrystalline cellulose, magnesium aluminum silicate, triethanolamine, polyvinyl alcohol (PVA), polyvinylpyrrolidone (PVP), 4-(1,1,3,3-tetramethylbutyl)-phenol polymer with ethylene oxide and formaldehyde (also known as tyloxapol, superione, and triton), poloxamers (e.g., Pluronics F68^{®} and F108^{®}, which are block copolymers of ethylene oxide and propylene oxide); poloxamines (e.g., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); a charged phospholipid such as dimyristoyl phophatidyl glycerol, dioctylsulfosuccinate (DOSS); Tetronic 15080 (T-1508) (BASF Wyandotte Corporation), dialkylesters of sodium sulfosuccinic acid (e.g., Aerosol OT^{®}, which is a dioctyl ester of sodium sulfosuccinic acid (American Cyanamid)); Duponol P^{®}, which is a sodium lauryl sulfate (DuPont); Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-10G^{®} or Surfactant 10-G^{®} (Olin Chemicals, Stamford, Conn.); Crodestas SL-40^{®} (Croda, Inc.); and SA9OHCO, which is C 18H 37CH 2(CON(CH 3)CH 2(CHOH) 4(CH 2OH) 2 (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-nonyl β-D-glucopyranoside; octanoyl-N-methylglucamide; n-octyl-µ-D-glucopyranoside; octyl β-D-thioglucopyranoside; and the like. Tyloxapol is a particularly preferred additive for the pulmonary or intranasal delivery of steroids, even more so for nebulization therapies.

Representative examples of additives include Cacao Butter, Rose Oil Extract, Pumpkin Seed Oil Extract, Griffonia Simplicifolia Oil Extract, Celandine Oil Extract, Chamomile Extract, Liquorice Extract, Pure vanilla extract.

Stable suspension of heterocrystals of titanium dioxide and particles of silicon dioxide is characterized by presence on the lattice surface of electronically-excited triplet oxygen ³O₂ in the energy centers, namely, in the quantum dots - zones of local overheating, ensuring catalytic activity for formation of active forms of oxygen in a living organism.

Polydispersity of obtained suspensions of heterocrystals of titanium dioxide and particles of silicon dioxide determines their unique properties at forming pharmaceutical compositions, that ensures not only targeted delivery into the area of pathological processes, but also unique mechanisms of action in their use.

As a result of the fact that the surface of crystals of titanium dioxide and particles of silicon dioxide contains the oxygen and presence in the ruptures of the developed surface of lattices of a great number of ionic groups of ligands formation of excitonic structures, ionic bonds and a range of zones of local energy overheating is ensured, all the types of sorption are possible, that determines detoxication possibilities of the obtained pharmaceutical compositions.

Presence on the crystal surface of oxygen O₂ presence in the ruptures of the developed surface of crystals lattices of a great number of ionic groups of ligands ensures formation of excitonic structures, ionic bonds and zones of local energy overheating, i.e. the quantum dots, where O₂ is in metastable electronically-excited triplet condition (²T³⁺) with unique characteristics of conversion into biological activity - singlet condition (S⁻¹⁻³).

It has been earlier demonstrated that in one dose of the preparation 1 mg/ml including stable suspension of titanium dioxide crystals or silicon dioxide particles their sorption capacity as a result of said sorption properties of crystals and particles is multiply higher in comparison with the existing preparations.

Peculiarity of the crystals and particles is that O₂ on their surface and is initially in excited triplet condition, with regulated possibility of catalysis in ROS, even in the cases where the human body temperature 36.6°C is sufficient, using in pharmaceutical compositions, which at the expense of synthesis of regulated active oxygen, ROS, firstly, selectively penetrate into the pathogenic cells of organism, targeting the areas of inflammation, with infectious and non-infectious nature, in particular, the cells using a ferment NADPH - for ROS formation, phagocytes and macrophages, causing in them numerous cytomorphological changes (vacuoles in cytoplasm, fragmentation of membrane, abnormality of mitosis), that launches apoptosis or necrosis type of death of pathogenic cells (because the pathogenic cells do not have effective antioxidant ferments).

NADFH - oxydases membrane-bound enzymatic complex, inversed into extracellular space of plasmatic membrane, and also in membranes of phagosomes, used by neutrophilic leukocytes (immune cells, white blood cells) for absorption of microorganisms is executed in the immune response.

Biocompatible TiO₂ crystals and SiO₂ particles are the only most indifferent in use in different forms of medical agents.

Laboratory and clinical researches, when oxygen, O₂, on the crystal lattices is initially in excited condition ²T³ in the case of the crystals-sensitizers use in composition of medicines hydrogels (forms of use: topically, in nose, in throat, in mucous layer, vaginally, rectally, orally), and also medical articles of topical use in the form of hydrocolloidal medical plasters, impregnation for tampons, absorbent pads, diapers, tapes, bandages etc., having antimicrobial and antiseptic influence, anti-inflammatory action, immunomodulatory and adsorbing action, depending on form and method of their use.

Pharmaceutical composition is used as adjuvant therapy, both independently and in combination with oncotherapy, antiviral, vaccination, for orthopaedic use.

In adjuvant therapy pharmaceutical composition is used independently or with active pharmaceutical ingredients and pharmaceutically acceptable additions.

Absence of general and local toxicity is confirmed by the results of toxicological researches.

The side effects in examinations of toxicity and examination of the repeated doses were not revealed, and the results of researches also confirm absence of mutagenicity and clastogenicity. Parenteral tolerated dose for TiO₂ is 21.72 mg/kg bw per day, and for SiO₂ 11.39 mg/kg bw per day. Peroral tolerated dose for TiO₂ is 2172 mg/kg bw per day, and for SiO₂ 1139 mg/kg bw per day (Dossier No. 488.729.2117; 488.729.2119 from 06.07.2010, TOXI COOP Zrt).

Stable suspension of titanium dioxide crystals and silicon dioxide particles ensures induction of immune response of a vertebrate by means of physical or chemical interaction with antigens.

Biological inertness of TiO₂ crystals and SiO₂ particles is well-known, and by their expressed and regulated biological activity they acquire in the course of a special method of their obtaining, as a result of which the local zones of energy overheating appear (de la Hoz A, Diaz-Ortiz A, Moreno A. Microwaves in organic synthesis. Thermal and non-thermal microwave effects. Chem Soc Rev. 2005 Feb;34(2):164-78. doi: 10.1039/b411438h. Epub 2005 Jan 12. PMID: 15672180), ensuring catalytic activity for formation of active forms of oxygen in a living organism.

Regulated active oxygen, ROS has become first demanded in the pathologies in focus (respiratory explosion), where oxygen necessity is multiplied.

Catalytic properties of crystals and particles are unique in the field of preparing pharmaceutical compositions for medical agents used for influence on a wide range of pathogens.

Photocatalytic properties of titanium dioxide or silicon dioxide heterocrystals - mobilizing at immune reaction to pathogens and normalization of ROS production in a living organism.

Hereinafter we present the examples of obtaining pharmaceutical compositions for restoring the physiological processes and cells of organism on the base of different cyclodextrins, chosen from the group of cyclomaltooligosaccharides, namely alpha-cyclodextrins, beta-cyclodextrins, gamma-cyclodextrins, randomly methylated beta-cyclodextrins, 2-hydropropyl-beta-cyclodextrins, sulfobutylated beta-cyclodextrins, 2-hydropropyl-gamma-cyclodextrins or their appropriate mixtures and sugammadex, in combination with aqueous solution of stable suspension of heterocrystals of titanium dioxide or particles of silicon dioxide with size no less than 450 nm having on the surface of crystals and particles in the energy centers electronically-excited triplet oxygen ³O₂.

Example 1. Obtaining and properties of aqueous nanodispersion of methylated beta-cyclodextrin (CD) and titanium dioxide or silicon dioxide.

Different amounts of randomly methylated beta-cyclodextrin (RAMEB, Wacker Chemie, Munich, Germany, CAVASOL^{®} W7 M 1.8 with molar substitution of 1.8 methyl group per a glucopyranose unit) were dissolved in 100 l of purified water at room temperature at slow, from 600 rotation/minute mixing. Obtained solutions were transparent and had pH between 6.2-6.5.

In another vessel suspension of activated crystals or particles TiO₂ or SiO₂ quantum dots (QD), PCT/IB2022/054063 was mixed in water for injection (WFI) in concentration up to 10% TiO₂ or in WFI in concentration up to 5% SiO₂, dispersed in 100 l of purified water (WFI) in the hydrodynamical cavitational homogenizer, for obtaining stable suspension of TiO₂ heterocrystals or SiO₂ particles in specified concentration.

Process of treatment in the hydrodynamical cavitational homogenizer lasts from 10 minutes depending on specified indices required for suspension.

According to the results of readiness of preliminary mixing in two separate vessels the CD solution and suspension of heterocrystals or particles, which were enough homogenous for subsequent homogenizing by the hydrodynamical cavitational homogenizer, PCT/IB2022/054057, where the process of homogenization is executed simultaneously with delivery from two vessels, one vessel as basic and one as dosed.

At the final phase the obtained pharmaceutical composition is filled into package from 1 g to 20 kg.

In the examples 2-31 the process of preparation of aqueous nanodispersion of cyclodextrins (2-Hydroxypropyl beta-cyclodextrin, Sulfobutylated beta-cyclodextrin sodium salt) and titanium dioxide or silicon dioxide or their mixture is analogous.

The variants of pharmaceutical compositions according to the examples 1-31 and their characteristic is shown in the Tables 1-3, the Tables 1-2 show suspensions of TiO₂ heterocrystals and SiO₂ particles, and the Table 3 shows variants of pharmaceutical compositions containing suspension of mixture of TiO₂ heterocrystals and SiO₂ particles in declared amount.

**Table 1**

| Example No. | Ingredient | Amount % mass | Ingredient | Amount % mass | Physical form | Characterized by ROS production |
|---|---|---|---|---|---|---|
| 1 | silicon dioxide (SiO₂) | 0.0001 | Alpha cyclodextrin (ACD) | 99.999 | Aqueous dispersion | Is produced |
| 2 | silicon dioxide (SiO₂) | 0,01 | Beta cyclodextrin (BCD) | 99.99 | Aqueous dispersion | Is produced |
| 3 | silicon dioxide (SiO₂) | 0,1 | Gamma cyclodextrin (GCD) | 3 | Aqueous dispersion | Is produced |
| 4 | silicon dioxide (SiO₂) | 2 | SBECD* | 5 | Aqueous dispersion | Is produced |
| 5 | silicon dioxide (SiO₂) | 5 | HPGCD** | 0.001 | Aqueous dispersion | Is produced |
| 6 | silicon dioxide (SiO₂) | 0.1 | Sugammadex | 0.5 | Aqueous dispersion | Is produced |
| 7 | silicon dioxide (SiO₂) | 0.001 | SBECD* | 99.99 | Aqueous dispersion | Is produced |
| 8 | silicon dioxide (SiO₂) | 3 | HPGCD** | 30 | Aqueous dispersion | Is produced |
| 9 | silicon dioxide (SiO₂) | 0.05 | Sugammadex | 0.95 | Aqueous dispersion | Is produced |
| 10 | silicon dioxide (SiO₂) | 0.99 | SBECD* | 1.00 | Aqueous dispersion | Is produced |
| 11 | silicon dioxide (SiO₂) | 0.1 | HPGCD** | 3.00 | Aqueous dispersion | Is produced |
| 12 | silicon dioxide (SiO₂) | 0.005 | Sugammadex | 0.095 | Aqueous dispersion | Is produced |

| | | | | | | |
|---|---|---|---|---|---|---|
| SBECD* sulfobutylated beta-cyclodextrin HPGCD** 2-hydroxypropyl-gamma-cyclodextrin | | | | | | |

**Table 2**

| Example No. | Ingredient | Amount % mass | Ingredient | Amount % mass | Physical form | Characterized by ROS production |
|---|---|---|---|---|---|---|
| 13 | titanium dioxide (TiO₂) | 0.0015 | Alpha cyclodextrin (ACD) | 99.985 | Aqueous dispersion | Is produced |
| 14 | titanium dioxide (TiO₂) | 0,01 | Beta cyclodextrin (BCD) | 99.99 | Aqueous dispersion | Is produced |
| 15 | titanium dioxide (TiO₂) | 0.3 | Gamma cyclodextrin (GCD) | 3 | Aqueous dispersion | Is produced |
| 16 | titanium dioxide (TiO₂) | 2 | SBECD* | 87 | Aqueous dispersion | Is produced |
| 17 | titanium dioxide (TiO₂) | 5 | HPGCD** | 0.001 | Aqueous dispersion | Is produced |
| 18 | titanium dioxide (TiO₂) | 1 | Sugammadex | 30 | Aqueous dispersion | Is produced |
| 19 | titanium dioxide (TiO₂) | 0.001 | SBECD* | 99.99 | Aqueous dispersion | Is produced |
| 20 | titanium dioxide (TiO₂) | 2 | HPGCD** | 30 | Aqueous dispersion | Is produced |
| 21 | titanium dioxide (TiO₂) | 0.5 | Sugammadex | 0.95 | Aqueous dispersion | Is produced |
| 22 | titanium dioxide (TiO₂) | 0.99 | SBECD* | 1.00 | Aqueous dispersion | Is produced |
| 23 | titanium dioxide (TiO₂) | 3 | HPGCD** | 3.00 | Aqueous dispersion | Is produced |
| 24 | titanium dioxide (TiO₂) | 0.55 | Sugammadex | 0.45 | Aqueous dispersion | Is produced |

| | | | | | | |
|---|---|---|---|---|---|---|
| SBECD* sulfobutylated beta-cyclodextrin HPGCD** 2-hydroxypropyl-gamma-cyclodextrin | | | | | | |

**Table 3**

| Example No. | Ingredient | Amou nt % mass | Ingredient | Amou nt % mass | Ingredien t | Amou nt % mass | Physical form | Characterize d by ROS production |
|---|---|---|---|---|---|---|---|---|
| 25 | silicon dioxide (SiO₂) | 3 | titanium dioxide (TiO₂) | 2 | SBECD* | 93.00 | Aqueous dispersion | Is produced |
| 26 | silicon dioxide (SiO₂) | 0.1 | titanium dioxide (TiO₂) | 0.1 | SBECD* | 99.98 | Aqueous dispersion | Is produced |
| 27 | silicon dioxide (SiO₂) | 0.001 | titanium dioxide (TiO₂) | 0.2 | SBECD* | 3 | Aqueous dispersion | Is produced |
| 28 | silicon dioxide (SiO₂) | 3 | titanium dioxide (TiO₂) | 0.3 | SBECD* | 5 | Aqueous dispersion | Is produced |
| 29 | silicon dioxide (SiO₂) | 0.05 | titanium dioxide (TiO₂) | 4.55 | SBECD* | 0.01 | Aqueous dispersion | Is produced |
| 30 | silicon dioxide (SiO₂) | 0.99 | titanium dioxide (TiO₂) | 2.3 | SBECD* | 0.5 | Aqueous dispersion | Is produced |
| 31 | silicon dioxide (SiO₂) | 0.1 | titanium dioxide (TiO₂) | 0.1 | SBECD* | 40.0 | Aqueous dispersion | Is produced |

| | | | | | | | | |
|---|---|---|---|---|---|---|---|---|
| SBECD* sulfobutylated beta-cyclodextrin | | | | | | | | |

From the Tables 1-3 it is evident that the pharmaceutical compositions containing the above-listed CD and suspensions of activated SiO₂ or/and TiO₂ particles ensure ROS production in all the declared quantitative values.

Measuring of active forms of oxygen (ROS) in the pharmaceutical compositions were executed by means of determination of stationary levels of prooxidants (hydroperoxides) at the cells HT-1080 of fibrosarcoma using sensitive to oxidation 5,6-carboxi-2,7-dichlordihydrofluorescein diacetate (CDCFH₂) 10 mg/ml. Its reactive-capable product fluorescent coloring agent was detected by the fluorescent microscope Zeiss or measured at the fluorescent device using bandpass filter with λ ex = 485 nm and λ em = 530 nm. Using Fluoroscan Ascent. Florida, Labsystems.

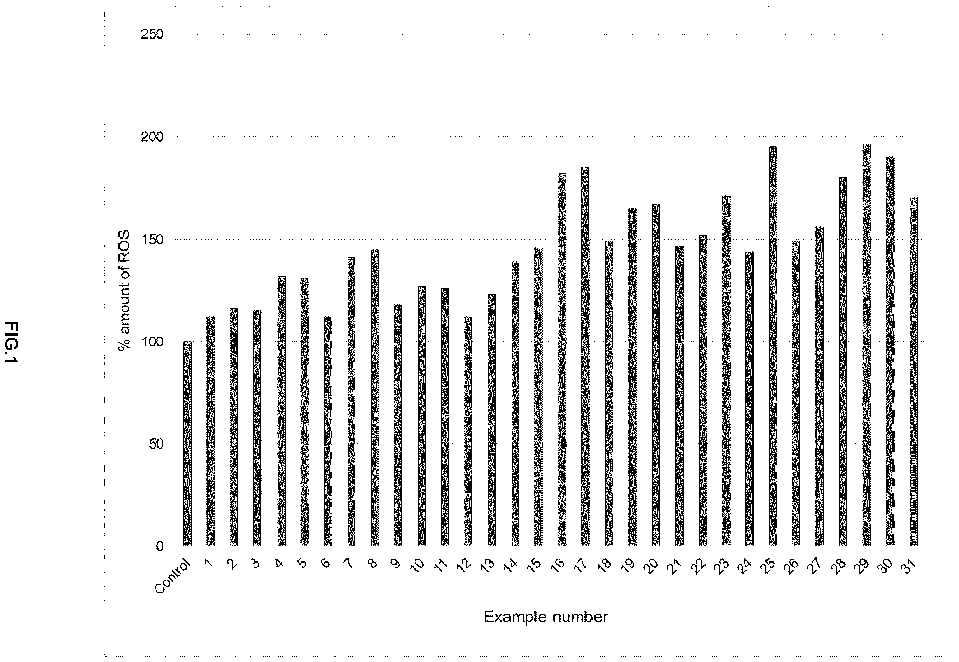

Results of researches are shown at the Fig.1, where dependence of ROS amount on the tested example of pharmaceutical composition is shown. On the x-axis - % amount of ROS on the y-axis - numbers of examples.

The results of measurements in the presented samples demonstrate a stable dependence on the amount and concentration of nanoparticles and cyclodextrins. The composition of TiO2, SiO2 with SBECD demonstrates a greater significance of ROS formation. Nevertheless, the formation of ROS in different amounts was confirmed in all samples.

The results of researches show that ROS production occurs in significant amount in comparison with a control sample (methylated beta-cyclodextrin).

### K-Control - 100 %

1. Pharmaceutical composition of the example 1 - 112 %
2. Pharmaceutical composition of the example 2 - 116 %
3. Pharmaceutical composition of the example 3 - 115 %
4. Pharmaceutical composition of the example 4 - 132 %
5. Pharmaceutical composition of the example 5 - 131 %
6. Pharmaceutical composition of the example 6 - 112 %
7. Pharmaceutical composition of the example 7 - 141 %
8. Pharmaceutical composition of the example 8 - 145 %
9. Pharmaceutical composition of the example 9 - 118 %
10. Pharmaceutical composition of the example 10 - 127 %
11. Pharmaceutical composition of the example 11 - 126 %
12. Pharmaceutical composition of the example 12 - 112 %
13. Pharmaceutical composition of the example 13 - 123 %
14. Pharmaceutical composition of the example 14 - 139 %
15. Pharmaceutical composition of the example 15 - 146 %
16. Pharmaceutical composition of the example 16 - 182 %
17. Pharmaceutical composition of the example 17 - 185 %
18. Pharmaceutical composition of the example 18 - 149 %
19. Pharmaceutical composition of the example 19 - 165 %
20. Pharmaceutical composition of the example 20 - 167 %
21. Pharmaceutical composition of the example 21 - 147 %
22. Pharmaceutical composition of the example 22 - 152 %
23. Pharmaceutical composition of the example 23 - 171 %
24. Pharmaceutical composition of the example 24 - 144 %
25. Pharmaceutical composition of the example 25 - 195 %
26. Pharmaceutical composition of the example 26 - 149 %
27. Pharmaceutical composition of the example 27 - 156 %
28. Pharmaceutical composition of the example 28 - 180 %
29. Pharmaceutical composition of the example 29 - 196 %
30. Pharmaceutical composition of the example 30 - 190 %
31. Pharmaceutical composition of the example 31 - 170 %

Hereinafter we present the examples of obtaining compositions of medical agents characterized by therapeutically significant effect for restoring the physiological processes and cells of organism.

Example 32. Obtaining and properties of aqueous nanodispersion of 2-hydroxypropylbeta-cyclodextrin and silicon dioxide in the form of hydrogel as pharmaceutical means for prophylaxis and therapy of ENT and viral diseases.

**Table 4**

| **Ingredient / Tradename** | **Pharmaceutical names** | **100,00 %** |
|---|---|---|
| **Phase I** | | |
| **Water** | WFI | Up to 100 % |
| **Natrosol 250 HHX Pharm** | Hydroxyethylcellulose | 1,50 % |
| **Water** | WFI | 10,00 % |
| **HPBCD** | 2 - Hydroxypropyl beta-cyclodextrin, HPBCD, Wacker Chemie, Munich, Germany, CAVASOL^{®} W7 HP PHARMA 4.2 substituents for cyclodextrin ring | 5,00 % |

| **Phase II** | | |
|---|---|---|
| **Potassium sorbate** | Potassium sorbate | 0,10 % |
| **Sodium benzoate** | Sodium benzoate | 0,5 % |
| **Polysorbate 80** | Polysorbate 80 | 2,00 % |
| **Vaseline oil** | Paraffinum Liquidum | 0,60 % |

| **Phase III** | | |
|---|---|---|
| **ADAM QD/S (0.6 mg/ml)** | Silicon Dioxide | 0,6 % |
| **Glycerol** | Glycerin | **3,00 %** |
| **Propyleneglycol** | Propylene Glycol | 4,00 % |
| **Menthol crystal** | Menthol | 0,5 % |

Composition of ingredients listed in the Table 4 in each phase of preliminary mixing was placed in three tank mixers and mixing was executed simultaneously.

According to the results of readiness of preliminary mixing in three tanks, the compositions that were enough homogenous for subsequent homogenizing were used for obtaining stable suspensions (hydrogel), by hydrodynamical cavitational homogenizer, where the homogenizing process was executed simultaneously with delivery from three tank mixers.

At the final phase the obtained mass of hydrogel, composition of medical agents is filled into package from 1 g up to 20 kg.

Medical agent of the Example 32 was characterized by the following methods:
Content of silicon dioxide in products was determined by ICP-analysis.

Average size of dispersed nanoparticles of silicon dioxide was in the range of 45-150 nm, as determined by Malvern Zetasizer Nano ZS.

Content of 2-hydroxypropyl-beta-cyclodextrin in the products was determined by HPLC.

Characteristic of the obtained ready composition has the following indices: - pH 5.5, transparent.

The final structure of hydrogel is: cyclodextrins 5 mass%, particles of silicon dioxide 0.6 mass%, pharmaceutically admissible additives 12.2 mass%, water for injections up to 100 mass%.

Example 33. Obtaining and properties of aqueous nanodispersion of 2-hydroxypropylbeta-cyclodextrin, and particles of titanium dioxide with obtaining gel for pharmaceutical medical agent for obtaining hydrogel, as pharmaceutical medical agent for prophylaxis and therapy of orthopedic, gynecological, urological, endocrinological, autoimmune, gastroenterological, neurologic, cardiological, oncological, dermatological, viral and ENT diseases.

**Table 5**

| **Ingredient / Tradename** | **Pharma names** | **100,00 %** |
|---|---|---|
| **Phase I** | | |
| **Water** | WFI | Up to 100 % |
| **Natrosol 250 HHX Pharm** | Hydroxyethylcellulose | 1,50 % |
| **Water** | WFI | 10,00 % |
| **HPBCD** | 2 - Hydroxypropyl beta-cyclodextrin, HPBCD, Wacker Chemie, Munich, Germany Munich, Germany, CAVASOL^{®} W7 HP PHARMA 4.2 substituents for cyclodextrin ring | 5,00 % |

| **Phase II** | | |
|---|---|---|
| **Potassium sorbate** | Potassium sorbate | 0,10 % |
| **Sodium benzoate** | Sodium benzoate | 0,5 % |
| **Polysorbate 80** | Polysorbate 80 | 2,00 % |
| **Vaseline oil** | Paraffinum Liquidum | 0,60 % |

| **Phase III** | | |
|---|---|---|
| **ADAM QD/T (0.6 mg/ml)** | Titanium Dioxide | 0,6 % |
| **Glycerol** | Glycerin | **3,00 %** |
| **Propylene glycol** | Propylene Glycol | 4,00 % |

Composition of ingredients listed in the Table 5 in each phase of preliminary mixing the ingredients was placed in three tank mixers and mixing was executed simultaneously.

According to the results of readiness of preliminary mixing in three tanks, the compositions that were adequately homogenous for subsequent homogenizing were used for obtaining stable suspensions (hydrogel), by hydrodynamical cavitational homogenizer, where the homogenizing process was executed simultaneously with delivery from three tank mixers.

At the second phase the obtained mass of hydrogel, composition of medical agents is filled into package from 1 g up to 20 kg.

Compositions of the Example 33 were characterized by the following methods:
Content of silicon dioxide in products was determined by ICP-analysis.

Average size of dispersed nanoparticles of silicon dioxide was in the range of 45-150 nm, as determined by Malvern Zetasizer Nano ZS.

Content of 2-hydroxypropyl-beta-cyclodextrin in the products was determined by HPLC.

Characteristic of the obtained ready composition has the following indices: pH 5.5, transparent.

The final structure of hydrogel is: cyclodextrins 5 mass%, crystals of titanium dioxide 0.6 mass%, pharmaceutically admissible additives 11.7 mass%, water for injections up to 100 mass%.

Example 34. Obtaining and properties of aqueous nanodispersion of 2-hydroxypropylbeta-cyclodextrin, and particles of titanium dioxide in the form of hydrogel as pharmaceutical medical agent for rejuvenation, prophylaxis and therapy of orthopaedic and cosmodermatological diseases.

**Table 6**

| **Ingredient /Tradename** | **Pharma names** | **100,00 %** |
|---|---|---|
| **Phase I** | | |
| **Water** | WFI | Up to 100 % |
| **HPBCD** | 2 - Hydroxypropyl beta-cyclodextrin, HPBCD, Wacker Chemie, Munich, Germany, CAVASOL^{®} W7 HP PHARMA 4.2 substituents for cyclodextrin ring | 1,00 % |
| **ADAM QD/T (0.6 mg/ml)** | Titanium Dioxide | 0,6 % |
| **Hyaluronic Acid** | Hyaluronic Acid, medical grade Stanford chemicals | 1% |

Composition of ingredients listed in the Table 6 in the first phase of preliminary mixing was placed in two tank mixers and mixing was executed simultaneously.

According to the results of readiness of preliminary mixing in two tank mixers, the compositions that were enough homogenous for subsequent homogenizing were used for obtaining stable suspensions (hydrogel), by hydrodynamical cavitational homogenizer, where the homogenizing process was executed simultaneously with delivery from two tank mixers, one mixture as basic and the second as dosed one.

At the second phase the obtained homogenous substance of the Example 34 is filled into package from 1 g up to 20 kg.

Compositions of the Example 34 were characterized by the following methods:
Content of silicon dioxide in products was determined by ICP-analysis.

Average size of dispersed nanoparticles of titanium dioxide was in the range of 45-150 nm, as determined by Malvern Zetasizer Nano ZS
Content of 2-hydroxypropyl-beta-cyclodextrin and Sulfobutylated beta-cyclodextrin in the products was determined by HPLC.

Characteristic of the obtained ready composition has the following indices: pH 5.5, transparent.

The final structure of hydrogel is: cyclodextrins 1.0 mass%, particles of titanium dioxide 0.6 mass%, pharmaceutically admissible additives 1.0 mass%, water for injections up to 100 mass%.

Example 35. Obtaining and properties of aqueous nanodispersion of sulfobutylated beta-cyclodextrin and the particles of silicon dioxide in the form of hydrogel, as pharmaceutical medical agent for prophylaxis and therapy of autoimmune, neurological, oncological and ENT diseases.

**Table 7**

| **Ingredient /Tradename** | **Pharma names** | **100,00 %** |
|---|---|---|
| **Phase I** | | |
| **Water** | WFI | Up to 100 % |
| **PVP** | polivinyl pyrrolidone | 3% |
| **SBECD** | Sulfobutylated beta-cyclodextrin sodium salt, Budapest, Hungary DEXOLVE^{®} pharma grade (DS∼6.5) | 0,30 % |
| **ADAM QD/S (0.6 mg/ml)** | Silicon Dioxide | 0,6 % |

Composition of ingredients listed in the Table 7 in the first phase of preliminary mixing was placed in two tank mixers and mixing was executed simultaneously.

According to the results of readiness of preliminary mixing in two tank mixers, the compositions that were enough homogenous for subsequent homogenizing were used for obtaining stable suspensions (hydrogel), by hydrodynamical cavitational homogenizer, where the homogenizing process was executed simultaneously with delivery of mixture as basic from one tank mixer, and the second as dosed one.

At the second phase the obtained homogenous substance of the Example 35 is filled into package from 1 g up to 20 kg.

Compositions of the Example 35 were characterized by the following methods:
Content of silicon dioxide in products was determined by ICP-analysis.

Average size of dispersed nanoparticles of silicon dioxide was in the range of 45-150 nm, as determined by Malvern Zetasizer Nano ZS.

Content Sulfobutylated beta-cyclodextrin in the products was determined by HPLC.

Characteristic of the obtained ready composition has the following indices: pH 5.5, transparent.

The final structure of hydrogel is: cyclodextrins 1.0 mass%, particles of silicon dioxide 0.6 mass%, pharmaceutically admissible additives 3.0 mass%, water for injections up to 100 mass%.

Example 36. Obtaining and properties of aqueous nanodispersion of 2-hydroxypropylbeta-cyclodextrin and silicon dioxide in the form of oil-water mixture, as pharmaceutical medical agent for prophylaxis and therapy of gynaecological, urological, orthopaedic, endocrinological, gastroenterological and viral diseases.

**Table 8**

| **Ingredient /Tradename** | **Pharma names** | **100,00 %** |
|---|---|---|
| **Phase I** | | |
| **White petroleum jelly** | Petrolatum | 9 % |
| **Cetostearyl alcohol** | Cetearyl Alcohol | 6 % |
| **Cacao Butter** | Theobroma Cacao Seed Butter | 1-5 % |
| **Oils** | Rose Oil Extract, Pumpkin Seed Oil Extract, Griffonia Simplicifolia Oil Extract, Celandine Oil Extract, Chamomile Extract, Liquorice Extract. | 1-7 % |

| **Phase II** | | |
|---|---|---|
| **ADAM QD/T-S(100mg/ml)** | Titanium Dioxide, Aqua (TiO₂ 10%, 100g/L) | 10,00 % |
| **PVP** | Polyvinylpyrrolidone | 3,00% |
| **Water** | WFI | 53,00 % |
| **HPBCD** | 2 - Hydroxypropyl beta-cyclodextrin, HPBCD, Wacker Chemie, Munich, Germany, CAVASOL^{®} W7 HP PHARMA 4.2 substituents for cyclodextrin ring | 0.5% |
| **Vanilla** | Pure vanilla extract | 0.5 % |
| **Natrosol 250 HHX Pharm** | Hydroxyethylcellulose | 1.5 % |

Composition of ingredients listed in the Table 8 in each phase of preliminary mixing was placed in three tank mixers and mixing was executed simultaneously.

In the first phase mixing of oil extracts and oil was executed, until obtaining enough homogenous mixture, for better homogeneity the homogenizer was used.

According to the results of readiness of preliminary mixing in tree tank mixers, the compositions that were enough homogenous for subsequent homogenizing were used for obtaining stable suspensions (aqueous-oil mixture), by hydrodynamical cavitational homogenizer, where the homogenizing process was executed simultaneously with delivery of mixture from one tank mixer, as basic one, and from two mixers as dosed ones.

At the second phase the obtained aqueous-oil mixture, composition of medical agents, is filled into package from 1 g up to 20 kg.

Compositions of the Example 36 were characterized by the following methods:
Content of silicon dioxide in products was determined by ICP-analysis.

Average size of dispersed nanoparticles of silicon dioxide was in the range of 45-150 nm, as determined by Malvern Zetasizer Nano ZS.

Content of 2-hydroxypropyl-beta-cyclodextrin in the products was determined by HPLC.

Characteristic of the obtained ready composition has the following indices: pH 5.5, transparent.

The final structure of hydrogel is: cyclodextrins 1.0 mass%, particles of silicon dioxide 0.6 mass%, pharmaceutically admissible additive 3.0 mass%, water for injections up to 100 mass%.

The final structure of aqueous-oil mixture is: cyclodextrins 0.50 mass%, particles of silicon dioxide 0.6 mass%, pharmaceutically admissible additives 89.5 mass%.

Example 37. Obtaining and properties of aqueous nanodispersion of 2-hydroxypropyl beta-cyclodextrin and silicon dioxide particles with obtaining lyophilized powder for pharmaceutical agent in the form of tablets, powders, capsules for prophylaxis ant therapy of orthopedic, gynecological, endocrinological, autoimmune, gastroenterological, neurological and viral diseases.

**Table 9**

| **Ingredient / Tradename** | **Pharma names** | **100,00 %** |
|---|---|---|
| **Phase I** | | |
| **Water** | WFI | Up to 100 % |
| **HPBCD** | 2 - Hydroxypropyl beta-cyclodextrin, HPBCD, Wacker Chemie, Munich, Germany, CAVASOL^{®} W7 HP PHARMA 4.2 substituents for cyclodextrin ring | 3,00 % |
| **ADAM QD/T (0.6 mg/ml)** | Titanium Dioxide | 0,6 % |

Compositions of ingredients listed in the Table 9 in the first phase of preliminary mixing was placed in two tank mixers and mixing was executed simultaneously.

According to the results of readiness of preliminary mixing in two tanks, the compositions that were enough homogenous for subsequent homogenizing were used, in particular, for obtaining stable suspensions by hydrodynamical cavitational homogenizer, where the homogenizing process was executed simultaneously with delivery of mixture from one tank as basic, and from another tank as dosed one.

At the second phase the obtained homogenous substance of the Example 37 was lyophilized in the vessel until total dehydration. Lyophilizing was executed at the expense of soft drying without use of high temperatures.

The obtained lyophilizate, composition (substance) in the form of powder for subsequent formation of medical agents in the form of tablets and capsules is filled into package from 1 g up to 1000 g.

Compositions of the Example 37 were characterized by the following methods:
Content of titanium dioxide in products was determined by ICP-analysis.

Average size of dispersed nanoparticles of silicon dioxide was in the range of 45-150 nm, as determined by Malvern Zetasizer Nano ZS.

Content of 2-hydroxypropyl-beta-cyclodextrin in the products was determined by HPLC.

Characteristic of the obtained ready composition has the following indices: white color powder.

The final structure of lyophilizate is: cyclodextrins 3.0 mass%, particles of titanium dioxide 0.6 mass%, water for injections up to 100 mass%.

Example 38. Obtaining and properties of aqueous nanodispersion of 2-hydroxypropylbeta-cyclodextrin and silicon dioxide, sulfobutylcyclodextrine and titanium dioxide particles in the form of lyophilized powder for pharmaceutical medical agent in the form of tablets, powders, capsules for prophylaxis ant therapy of orthopaedic, gynaecological, endocrinological, autoimmune, gastroenterological, neurological and viral diseases.

**Table 10**

| **Ingredient / Tradename** | **Pharma names** | **100,00 %** |
|---|---|---|
| **Phase I** | | |
| **Water** | WFI | Up to 100% |
| **HPBCD** | 2 - Hydroxypropyl beta-cyclodextrin, HPBCD, Wacker Chemie, Munich, Germany, CAVASOL^{®} W7 HP PHARMA 4.2 substituents for cyclodextrin ring | 3,00 % |
| **SBECD** | Sulfobutylated beta-cyclodextrin sodium salt, Budapest, Hungary DEXOLVE ^{®} pharma grade(DS~6.5) | 3,00 % |
| | **Phase II** | |
| **ADAM QD/T (0.6 mg/ml)** | Titanium Dioxide | 0,6 % |

Compositions of ingredients listed in the Table 10 in the first phase of preliminary mixing was placed in three tank mixers and mixing was executed simultaneously.

According to the results of readiness of preliminary mixing in three tanks, the compositions that were enough homogenous for subsequent homogenizing were used, in particular, for obtaining stable suspensions by hydrodynamical cavitational homogenizer, where the homogenizing process was executed simultaneously with delivery of mixture from one tank as basic, and from two tanks as dosed ones.

At the second phase the obtained homogenous substance of the Example 38 was lyophilized in the vessel until total dehydration. Lyophilizing was executed at the expense of soft drying without use of high temperatures.

The obtained lyophilizate, composition (substance) in the form of powder for subsequent formation of medical agents in the form of tablets and capsules is filled into package from 1 g up to 1000 g.

Compositions of the Example 38 were characterized by the following methods:
Content of silicon dioxide in products was determined by ICP-analysis.

Average size of dispersed nanoparticles of silicon dioxide was in the range of 45-150 nm, as determined by Malvern Zetasizer Nano ZS.

Content of 2-hydroxypropyl-beta-cyclodextrin and Sulfobutylated beta-cyclodextrin in the products was determined by HPLC.

Characteristic of the obtained ready composition has the following indices: white color powder.

The final structure of lyophilizate is: cyclodextrins 6.0 mass%, crystals of titanium dioxide 0.6 mass%, water for injections up to 100 mass%.

Example 39. Obtaining and properties of aqueous nanodispersion of Sulfobutylated beta-cyclodextrin and silicon dioxide particles in the form of lyophilized powder for pharmaceutical medical agent - tablets, powders, capsules for prophylaxis ant therapy of orthopaedic, gynaecological, endocrinological, autoimmune, gastroenterological, neurological and viral diseases.

**Table 11**

| **Ingredient / Tradename** | **Pharma names** | **100,00 %** |
|---|---|---|
| **Phase I** | | |
| **Water** | WFI | Up to 100% |
| **SBECD** | Sulfobutylated beta-cyclodextrin sodium salt, Budapest, Hungary DEXOLVE ^{®} pharma grade(DS~6.5) | 3,00 % |
| **ADAM QD/S (0.6 mg/ml)** | Silicon Dioxide | 0,6 % |

Compositions of ingredients listed in the Table 11 in the first phase of preliminary mixing was placed in three tank mixers and mixing was executed simultaneously.

According to the results of readiness of preliminary mixing in two tanks, the compositions that were enough homogenous for subsequent homogenizing were used, in particular, for obtaining stable suspensions by hydrodynamical cavitational homogenizer, where the homogenizing process was executed simultaneously with delivery of mixture from one tank as basic, and another as dosed one.

At the second phase the obtained homogenous substance of the Example 39 was lyophilized in the vessel until total dehydration. Lyophilizing was executed at the expense of soft drying without use of high temperatures.

The obtained lyophilizate (substance) in the form of powder for subsequent formation of medical agents in the form of powders, tablets and capsules is filled into package from 1 g up to 1000 g.

Compositions of the Example 39 were characterized by the following methods:
Content of silicon dioxide in products was determined by ICP-analysis.

Average size of dispersed nanoparticles of silicon dioxide was in the range of 45-250 nm, as determined by Malvern Zetasizer Nano ZS.

Content of sulfobutyl-beta-cyclodextrin in the products was determined by HPLC.

Characteristic of the obtained ready composition has the following indices: white color powder.

The final structure of lyophilizate is: cyclodextrins 3.0 mass%, particles of silicon dioxide 0.6 mass%, water for injections up to 100 mass%.

Example 40. Obtaining and properties of aqueous nanodispersion of 2-hydroxypropyl beta-cyclodextrin and silicon dioxide particles in the form of stable suspension as pharmaceutical medical agent for prophylaxis and therapy of endocrinological diseases.

**Table 12**

| **Ingredient Tradename** | **Pharma names** | **100,00 %** |
|---|---|---|
| **Phase I** | | |
| **Water** | WFI | Up to 100 % |
| **PVP** | polivinyl pyrrolidone | 3% |
| **HPBCD** | 2 - Hydroxypropyl beta-cyclodextrin, HPBCD, Wacker Chemie, Munich, Germany, CAVASOL^{®} W7 HP PHARMA 4.2 substituents for cyclodextrin ring | 0,10 % |
| **ADAM QD/S (0.6 mg/ml)** | Silicon Dioxide | 0,6 % |

Compositions of ingredients listed in the Table 12 in the first phase of preliminary mixing was placed in two tank mixers and mixing was executed simultaneously.

According to the results of readiness of preliminary mixing in two tank mixers, the compositions that were enough homogenous for subsequent homogenizing were used, in particular, for obtaining stable suspensions, by hydrodynamical cavitational homogenizer, where the homogenizing process was executed simultaneously with delivery of mixture from one tank mixer as basic and another as dosed one.

At the second phase the obtained quasistable suspension, composition of medical agents is filled into package from 1 ml up to 1000 ml.

Compositions of the Example 40 were characterized by the following methods:
Content of silicon dioxide in products was determined by ICP-analysis.

Average size of dispersed nanoparticles of silicon dioxide was in the range of 45-150 nm, as determined by Malvern Zetasizer Nano ZS.

Content of 2-hydroxypropyl-beta-cyclodextrin in the products was determined by HPLC.

Characteristic of the obtained ready composition has the following indices: pH 5.5, whitish color.

The final structure of suspension is: cyclodextrins 0.10 mass%, particles of silicon dioxide 0.6 mass%, pharmaceutically admissible additives 3.0 mass%, water for injections up to 100 mass%.

Example 41. Obtaining and properties of aqueous nanodispersion of 2-hydroxypropyl beta-cyclodextrin and titanium dioxide crystals in the form of stable suspension as pharmaceutical medical agent for prophylaxis ant therapy of orthopedic, urological, gynecological, endocrinological, autoimmune, gastroenterological, neurological, viral and dermatological diseases.

**Table 13**

| **Ingredient / Tradename** | **Pharma names** | **100,00 %** |
|---|---|---|
| **Phase I** | | |
| **Water** | WFl | Up to 100 % |
| **PVP** | polivinyl pyrrolidone | 3% |
| **HPBCD** | 2 - Hydroxypropyl beta-cyclodextrin, HPBCD, Wacker Chemie, Munich, Germany, CAVASOL^{®} W7 HP PHARMA 4.2 substituents for cyclodextrin ring | 0,30 % |
| **ADAM QD/T (0.6 mg/ml)** | Titanium Dioxide | 0,6 % |

Compositions of ingredients listed in the Table 13 in the first phase of preliminary mixing was placed in two tank mixers and mixing was executed simultaneously.

According to the results of readiness of preliminary mixing in two tanks, the compositions that were enough homogenous for subsequent homogenizing were used, in particular, for obtaining stable suspensions by hydrodynamical cavitational homogenizer, where the homogenizing process was executed simultaneously with delivery of mixture from one tank as basic and another as dosed one.

At the second phase the obtained quasistable suspension, composition of medical agents is filled into package from 1 ml up to 1000 ml.

Compositions of the Example 41 were characterized by the following methods:
Content of silicon dioxide in products was determined by ICP-analysis.

Average size of dispersed nanoparticles of titanium dioxide was in the range of 45-150 nm, as determined by Malvern Zetasizer Nano ZS.

Content of 2-hydroxypropyl-beta-cyclodextrin in the products was determined by HPLC.

Characteristic of the obtained ready composition has the following indices: pH 5.5, whitish color.

The final structure of suspension is: cyclodextrins 0.30 mass%, crystals of titanium dioxide 0.60 mass%, pharmaceutically admissible additives 3.0 mass%, water for injections up to 100 mass%.

Example 42. Obtaining and properties of aqueous nanodispersion of sulfobutylether beta-cyclodextrin and silicon dioxide particles in the form of stable suspension as pharmaceutical medical agent for rejuvenation, prophylaxis and therapy of orthopedic, cosmodermatological, dermatological diseases.

**Table 14**

| **Ingredient / Tradename** | **Pharma names** | **100,00 %** |
|---|---|---|
| **Phase I** | | |
| **Water** | WFl | Up to 100% |
| **PVP** | polivinyl pyrrolidone | 3% |
| **SBECD** | Sulfobutylated beta-cyclodextrin sodium salt, Budapest, HungaryHungary DEXOLVE^{®} pharma grade (DS∼6.5) | 0,10 % |
| **ADAM QD/S (0.6 mg/ml)** | Silicon Dioxide | 0,6 % |

Compositions of ingredients listed in the Table 14 in the first phase of preliminary mixing was placed in two tank mixers and mixing was executed simultaneously.

According to the results of readiness of preliminary mixing in two tank mixers, the compositions that were enough homogenous for subsequent homogenizing were used, in particular, for obtaining stable suspensions by hydrodynamical cavitational homogenizer, where the homogenizing process was executed simultaneously with delivery of mixture from one tank mixers as basic composition and from another as dosed one.

At the second phase the obtained homogenous substance of the Example 42 is filled into package from 1 g up to 20 kg.

Compositions of the Example 42 were characterized by the following methods:
Content of silicon dioxide in products was determined by ICP-analysis.

Average size of dispersed nanoparticles of silicon dioxide was in the range of 45-250 nm, as determined by Malvern Zetasizer Nano ZS.

Content of Sulfobutylated beta-cyclodextrin in the products was determined by HPLC.

Characteristic of the obtained ready composition has the following indices: pH 5.5, whitish suspension.

The final structure of suspension is: cyclodextrins 0.10 mass%, particles of silicon dioxide 0.6 mass%, pharmaceutically admissible additives 3.0 mass%, water for injections up to 100 mass%.

Example 43. Obtaining and properties of aqueous nanodispersion of sulfobutylether beta-cyclodextrin, silicon dioxide particles and Doxorubicin in the form of suspension as pharmaceutical medical agent of therapy of oncological diseases.

**Table 15**

| **Ingredient / Tradename** | **Pharma names** | **100,00 %** |
|---|---|---|
| **Phase I** | | |
| **Water** | WFI | Up to 100% |
| **SBECD** | Sulfobutylated beta-cyclodextrin sodium salt, Budapest, Hungary DEXOLVE ^{®} pharma grade (DS∼6.5) | 0,10% |
| **ADAM QD/S (0.6 mg/ml)** | Silicon Dioxide | 0,6 % |
| **Doxorubicin** | Doxorubicin Ebeve Austria powder | 0.001 % |

Compositions of ingredients listed in the Table 15 in the first phase of preliminary mixing was placed in two tank mixers and mixing was executed simultaneously.

According to the results of readiness of preliminary mixing in two tank mixers, the compositions that were enough homogenous for subsequent homogenizing were used, in particular, for obtaining stable suspensions by hydrodynamical cavitational homogenizer, where the homogenizing process was executed simultaneously with delivery of mixture from one tank as basic and another as dosed one.

At the second phase the obtained homogenous substance of the Example 43 is filled into package from 1 g up to 20 kg.

Compositions of the Example 43 were characterized by the following methods:
Content of silicon dioxide in products was determined by ICP-analysis.

Average size of dispersed nanoparticles of silicon dioxide was in the range of 45-150 nm, as determined by Malvern Zetasizer Nano ZS.

Content of Sulfobutylated beta-cyclodextrin in the products was determined by HPLC.

Characteristic of the obtained ready composition has the following indices: pH 5.5, transparent.

The final structure of suspension is: cyclodextrins 0.10 mass%, particles of silicon dioxide 0.6 mass%, pharmaceutically admissible additives 0.001%, water for injections up to 100 mass%.

Example 44. Obtaining and properties of Pharmaceutical composition (1) in the form of hydrogel or hydrocolloidal medical products, having in their structure cyclodextrins SBECD, HPBCD 0.5 mg/g, and TiO₂ crystals or SiO₂ particles, QD, 0.005 mg in 1 gram of hydrogel or hydrocolloidal composition.

### Method of application on the plaster:

Starting material of different necessary components ChinMed Technologies are submerged in the mixer, the mass is heated at the temperature from 190°C, after homogenizing a pharmaceutical composition is injected, comprising stable suspension of TiO₂ crystals or SiO₂ particles taken in concentration 0.0001 - 1.0% and cyclodextrins SBECD or HPBCD taken in concentration 0.0001 - 1.0%. After mixing sterilization occurred. At the output the obtained soft homogenous mass was reprocessed into a roll band from two sides, wound into a roll paper adding in the surface part a polyurethane (PU) adhesive for isolation of stickiness, and then it was delivered to the final forming and package.

Used pharmaceutical composition in the form of articles has antimicrobial and antiseptic influence, anti-inflammatory action, immunomodulatory and adsorbing action, depending on form and way of use.

Pharmaceutical composition in the structure of hydrocolloidal mass of a medical product, plaster, is in quasi-stable condition of crystals and particles, mechanism of action is that at the moment of contact with skin or mucous a thermal reaction occurs, resulting in accumulation of energy, obtained from superficially saturated with energy reactogenic centers crystals and particles of a special class of oxides (TiO₂, SiO₂), being semiconductors, the active molecules of oxygen are produced. Demonstration of oxygen activity promotes energy appearance and, correspondingly, transfer of reverse energy onto specified body areas, and as a result additional energy promotes enzyme complex NADPH, in conditions of increased need, initiation of additional ROS production in pathogenic focuses with therapeutic purposes, and also promotes normalization of functioning of neuromediator receptors of organism. So, on the example of key monoamine neuromediator serotonin, regulation of inhibition of reverse capture of serotonin occurs by the way of selective blocking of serotonin absorption by a membrane of presynaptic cell. Due to elimination of disbalance of neuromediator in synaptic gap neurotransmission intensifies in serotoninergic synapses.

Ensuring of balanced level of neuromediators in an organism has evident positive effect on the state of health of a human and promotes elimination of symptoms of different pathological conditions. And healthy functioning of serotonin receptors in an organism also assists to restore homeostasis quickly, promotes general health improvement and improvement of activity of the principal organs and systems of organism.

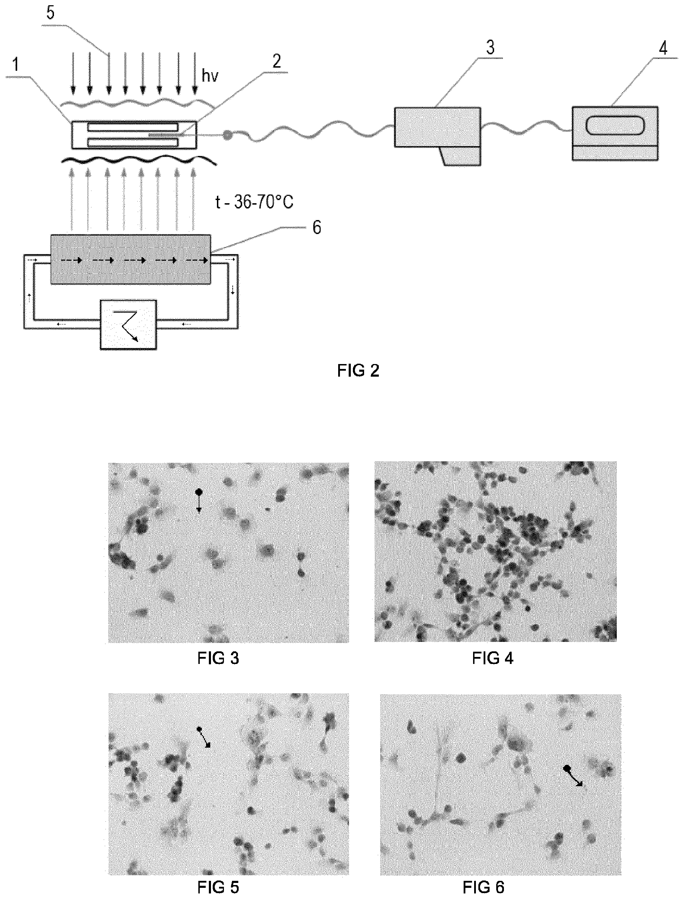

Formation of active form of oxygen is detected by the system shown at the Fig.2, where:
1. Hydrogel and hydrocolloidal medical products comprising pharmaceutical composition, containing cyclodextrins SBECD, HPBCD in quantity 0.0001 - 1.0% and TiO₂ crystals or SiO₂ particles, QD in quantity 0.0001 - 1.0% in 1 gram of hydrogel or hydrocolloidal mass.
2. The fiber optical sensor of oxygen oxides with sensitive coating in the form of a solid body for control of concentration S'O₂₊, of active form of oxygen (ROS).
3. Analyser of O₂, model FOMS - 200 OXYGEN.
4. PC for ensuring visualization of results in online mode.
5. Spectrum of radiation of IR-laser with radiation of 960 nm, or IR-laser.
6. The heater with circulation of warm water (stably 36.6°C).

The pharmaceutical composition (1) in the form of hydrogel in the structure of medical product containing cyclodextrins SBECD, HPBCD in quantity of 0.5 mg/g and TiO₂ crystals or SiO₂ particles, QD, in quantity of 0.005 mg in 1 gram of hydrogel or hydrocolloidal mass, is placed in a medium to which the sensor for registration (2) transmitting the signals to the analyzer (3) and PC (4), source of thermal influence (5) in the radiation spectrum of IR-laser with wavelength of 960 nm, or IR-laser, (6) heater with circulation of warm water (stably 36.6°C) are connected.

Peculiarity of the pharmaceutical composition is that regulated catalysis in ROS occurs at human body temperature 36.6°C, by means of photo- or thermo- catalytic induction of O₂ on the surface of crystals or particles of the pharmaceutical composition.

Examination of onco-biological effects of pharmaceutical compositions: cyclodextrins and TiO₂ and SiO₂ suspensions.

From the data of literature is known, that TiO₂ and SiO₂ have ability to reduce tumours volume in vivo (Behnam, Mohammad Ali et al. "The application of titanium dioxide (TO2) nanoparticles in the photo-thermal therapy of melanoma cancer model." Iranian journal of basic medical sciences vol. 21,11 (2018): 1133-1139. doi:10.22038/IJBMS.2018.30284.7304), and cyclodextrins have ability to improve effect of a therapeutical agent (Bai, H., Wang, J., Phan, C.U. et al. Cyclodextrin-based host-guest complexes loaded with regorafenib for colorectal cancer treatment. Nat Commun 12, 759 (2021). https://doi.org/10.1038/s41467-021-21071-0).

The results stated below demonstrate improvement of antitumoral activity of pharmaceutical compositions due to compositions of cyclodextrins and stable dispersions ADAM QD/T, TiO₂ and ADAM QD/S, which modulate protective mechanism of tumour cells.

A range of the experimental data showed a critical role of active forms of oxygen (ROS) and accompanying metabolic processes, including activation of signalling pathways, in cytotoxic action of titanium dioxide and silicon dioxide.

### Essence of examination

Demonstration of antitumoral effect of pharmaceutical compositions consisting of cyclodextrins and stable dispersion ADAM QD/T, TiO₂ and ADAM QD/S, SiO₂ in local administration.

A range of the experimental data on used pharmaceutical compositions shows at a critical role of active forms of oxygen (ROS) and accompanying metabolic processes, including activation of signalling pathways, in cytotoxic action of titanium dioxide and silicon dioxide. Therefore, importance of the mechanism of cellular response to generated ROS was proposed as a basic element in this research.

### Comparative agents:

### Compounds TiO₂ and SiO₂.

- TiO₂ (C YL- 3015) 3 mg/5 ml, 0.6 mg / ml;
- SiO₂ (CYL-3263) 3 mg /5 ml, 0.6 mg / ml;
- Doxorubicin (CYL-3251) 0.2 mg / ml;
- SiO₂- Doxorubicin (CYL-3252) 0.6 mg / ml; 0,2 mg / ml;
- HPBCD (CYL-3264) 5 mg / ml;
- TiO₂- HPBCD (CYL-3265) 0.6 mg /ml; 5 mg / ml;
- SiO₂ - SBECD - Doxorubicin (CYL-3261) 0.6 mg/ml; 5 mg/ml 0,2 mg/ml.

### Murine and human tumour cell lines:

A. ZR-75.1 Her-2 expressing human breast adenocarcinoma cell line.
B. HT-1080: H-ras expressing, producing matrix metalloprotease 2,9 human fibrosarcoma cell line,
C. Daoy Medulloblastoma cell line

### Cytomorphological researches

In these studies cells (5×10⁴) were seeded in 6-well plates and cultured in RPMI-1640 medium containing 10 % fetal bovine serum. The cultures were incubated for 2 hours in CO₂ thermostate at 37°C. After the preincubation period the medium was removed, the cells were washed with physiological saline (2 × 1ml 0.9% NaCl) and exposed to testing samples applying various concentrations for 60 min. After removing the test compounds fresh RPMI 1640 medium containing 10 % FBS was added and the cultures were incubated for further 24 hours at 37°C in humidified thermostate (5% CO₂). At the end of the posttreatment period the medium as supernatants of the cultures, containing released cells was removed and saved for cell counting.

### Analysis of tumour growth in vivo

### Colon38 murine model

Fragments of adenocarcinoma of murine large intestine were transplanted subcutaneously to male mice of the line C57BL. Treatments were executed 10-12 days after transplantation, when the tumour mass was about 0.2-0.4 g. Skin above the tumour was shaved and the tested compounds were administered immediately into the tumours. Treatment in aforesaid doses in calculation of mass mcg/tumour was executed intratumorally.

Changes in tumour growth were estimated controlling the tumour volume and measuring tumours mass at the moment of finishing the experiments. For evaluation of growth speed the tumour volume 24-26 days after transplantation was divided by the tumour volume measured during the first treatment (Vₜ/V₀). In this period tumours were in quickly growing condition.

### Model of xenotransplantant of a human tumour

The tumour cells HT1080 and ZR-75.1, obtained from the cell cultures, were transplanted orthotopically in amount of 2×10⁶ to naked mice. The tumours volumes were measured as described above. *The test substances* were administered inside the tumour, as indicated at the experimental area.

The data was expressed as average +/- standard deviation.

### The obtained results

### Cytotoxicity of test substances.

### Researches on Daoy medulloblastoma cell.

In this research the cytotoxic effects of TiO₂ (CYL-3015), SiO₂ (CYL-3263), HPBCD (CYL-3264); TiO₂ - HPBCD (CYL-3265) were compared. The cells of medulloblastoma revealed high sensitivity to the *test substances* at indicated concentrations. Different cytomorphological changes were observed: Arrows indicate changes as a result of the impact of tests on samples, particles near vacuolization of cytoplasm on Fig 3, appearance of multinuclear and giant cells on Fig 4, death of cells in the form of apoptotic corpuscles on Fig 5 and 6. The test substances TiO₂ (CYL-3015), TiO₂ - HPBCD (CYL-3265) themselves caused sharp damage of cells (Fig. 3-6).

### Researches on the cells culture HT1080

In subsequent experiments compounds of Doxorubicin (CYL-3251) and - SiO₂ - Doxorubicin (CYL-3252), SiO₂ - SBECD - Doxorubicin (CYL-3261), TiO₂ (C YL- 3015) and TiO₂ - HPBCD were examined in different compounds in order to make clear whether is possible to eliminate toxicity from Doxorubicin with saving cytotoxic action associated with SBECD, HPBCD, TiO₂, SiO₂. In administering of Doxorubicin only, i.e. without other components, evident cytotoxicity of Doxorubicin was observed at starting concentration.

In administering of compositions TiO₂ - HPBCD, when TiO₂ concentration was 600 mcg/ml, and cyclodextrin 5000 mcg/ml, cytotoxicity was noted.

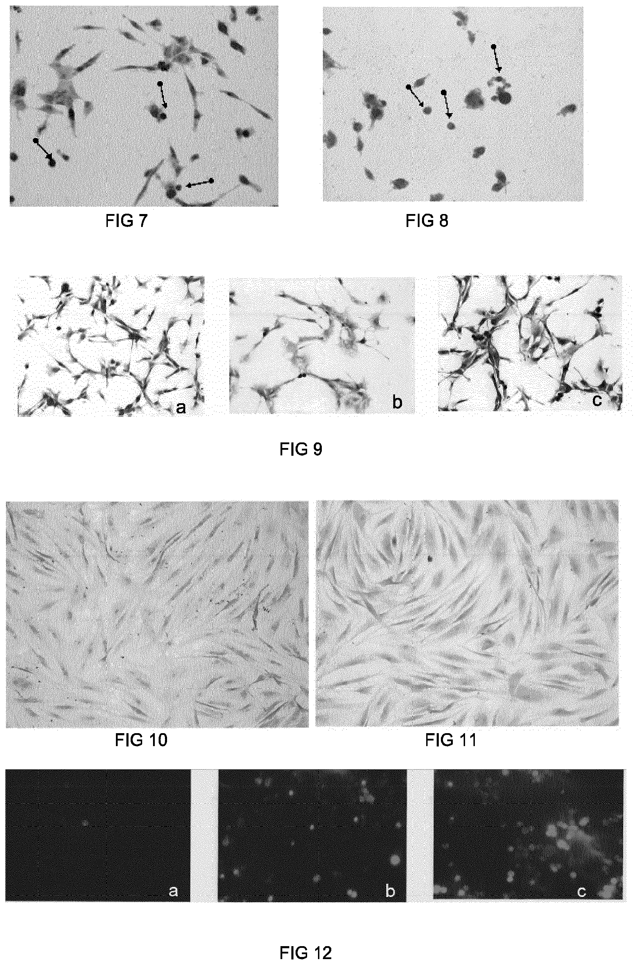

In administering of SiO₂ (CYL-3263), evident cytotoxicity was observed at concentration of 600 mcg/ml. Moreover, Doxorubicin in concentration 200 mcg/ml showed significant cytotoxicity. In use of SiO₂ - SBECD - Doxorubicin (CYL-3261) it is necessary to note increase of efficiency of compound at SiO₂ concentration 600 mcg/ml, SBECD 5000 5000 mcg/ml and Doxorubicin in concentration 200 mcg/ml and caused more evident and specific cytotoxicity (Fig. 7-8).

The research results confirmed antitumoral efficiency of pharmaceutical compositions SiO₂ - Doxorubicin (CYL-3252), SiO₂ - SBECD - Doxorubicin (CYL-3261) for transformed tumoral lines of cells, at the Fig. 9b and Fig. 9c structural morphological changes in comparison with the control Fig. 9a are evident, when the normal human fibroblasts were not affected (see: Fig. 10 and 11.)

*Critical metabolic processes for cytotoxic efficiency of pharmaceutical compositions TiO₂ (CYL- 3015); SiO₂ (CYL-3263);* - Doxorubicin *(CYL-3251); SiO₂* - Doxorubicin *(CYL-3252); HPBCD (CYL-3264); TiO₂* - *HPBCD (CYL-3265); SiO₂* - *SBECD* - Doxorubicin *(CYL-3261) were studied.*

Taking into account a conception that a better understanding of the mechanism of antitumoral action of examined samples could give recommendations for creation of efficient new therapeutic protocol, it was decided to characterize active forms of oxygen as supposed target for TiO₂ and SiO₂ in tumour cells. Therefore, antitumoral efficiency of TiO₂ and SiO₂ may be expected from revealing the cell processes determining whether treatment will result in death or survival of tumour cells.

In this research reinforced ROS formation showed dependence on combinations of test substances. So, *SiO₂* - *SBECD* - Doxorubicin *(CYL-3261)* produced greater amount of ROS in comparison with the test sample SiO₂ - Doxorubicin Fig. 12a control, Fig. 12b SiO₂ - Doxorubicin, Fig. 12c *SiO₂* - *SBECD* - Doxorubicin.

### Antitumoral action of test substances in vivo

Taking into account significant, expressed antitumoral activity of TiO₂ and SiO₂ nanoparticles in vivo, it is necessary to understand the optimal conditions for its efficiency.

*The role of speed of tumour growth in antitumoral action of pharmaceutical compositions.*

Since a size of transplanted fragment of tumour defines speed of the tumour growth, efficiency of TiO₂ - HPBCD (CYL-3265) and SiO₂ - SBECD - Doxorubicin were compared in two experimental groups with transplanted tumour fragments Colon 38 with small or big size. During therapy it was a significant difference in tumours volume (V₀) between two experimental groups. Speed of growth during subsequent 10 days was 2 times higher for animals with transplanted small tumour fragments, in comparison with the animals with transplanted big tumour fragments. Estimation of changes in tumour volume showed enough convincing proofs of depending on speed of growth efficiency of administered tested substances, since TiO₂ - HPBCD (CYL-3265) and SiO₂ - SBECD - Doxorubicin (CYL-3261) inhibit tumours with high speed of growth. The experiment showed that TiO₂ - HPBCD and SiO₂ - SBECD - Doxorubicin by 30% and 50% correspondingly suppress growth of transformed cells in culture Colon 38 in comparison with control.

### Researches on the model of xenotransplantant of a human tumour

In was a big problem to examine action of TiO₂ - HPBCD (CYL-3265) and SiO₂ - SBECD - Doxorubicin in vivo on tumours ZR.75.1 and HT1080, which showed remarkable sensitivity in cellular cultures. It was marked that in intratumoural administering in concentrations causing cytotoxicity in the cell cultures, changes in growth of the same tumour in the model of human xenotransplantant were not registered.

Thereby, the conclusion were made:
1.) TiO₂ - HPBCD (CYL-3265) causes serious cytomorphological changes, SiO₂ (CYL-3263) in lesser degree. In whole, in the cells reduced microvilluses, fragmented membranes, vacuoles, aberrant organization of nucleus, including apoptotic corpuscles and necrotic cells were observed.
   TiO₂ (CYL-3263) induced damage of the cells in such low concentration as 3 mg/ml.
2.) Doxorubicin (CYL-3251) showed moderate cytotoxicity 0.2 mg/ml. HPBCD (CYL-3264) 5 mg/ml did not show cytotoxic action. TiO₂ - HPBCD (CYL-3264), SiO₂ - Doxorubicin (CYL-3252) and SiO₂ - SBECD - Doxorubicin (CYL-3261) showed cytotoxicity.
3.) In comparison of cytomorphology of four lines of the human cells, the cells of medulloblastoma Daoy proved to be the most sensitive, and fibroblasts did not show signs of cellular damage.
4.) TiO₂ - HPBCD (CYL-3265) and SiO₂ - SBECD - Doxorubicin is efficient in quickly growing tumours, but in slowly growing tumours the tests are necessary during longer period of researches, than it is executed in the present research.
5.) TiO₂ - HPBCD (CYL-3265) and SiO₂ - SBECD - Doxorubicin enlarged the life duration of naked mice with tumour HT1080 without reducing the tumour volume, where it is also necessary to enlarge terms of research (because in the initial period of researches the tumour keeps its volume in the period of so-called therapeutic inflammation).
6.) The pharmaceutical compositions SiO₂ - SBECD- Doxorubicin (CYL-3261), TiO₂ - HPBCD (CYL-3265), and cytostatics (Doxorubicin, Lenalidomide (Revlimid), Nivolumab, Imbrutinib) showed positive effect on the model of metastasing into spleen and liver, having significantly reduced the initial tumour after intra-abdominal administration, before administration of cytostatic. Moreover, a tendency to decrease of metastatic focuses was observed.

The new pharmaceutical compositions TiO₂ - HPBCD, SiO₂ - SBECD - Doxorubicin in presented form demonstrate additional advantages over initial tested substances considering cytomorphological changes. Therefore, the indicated pharmaceutical compositions may be used for ensuring antitumoral activity as an adjuvant.

The presented experiments gave the additional data on variability of antitumoral efficiency of the pharmaceutical compositions depending on biological analysis, i.e. in vitro or in vivo, type of cellular population and presence of biological substrates.

The pharmaceutical compositions presented in the examples ensure ROS formation in damaged cells. ROS induce both physiological processes and molecular mechanisms, resulting in death of pathogenic cells.

Influence of pharmaceutical compositions on restoration of physiological processes, rejuvenation and life duration of Caenorhabditis Elegans.

The results of the research stated below demonstrate ability of used pharmaceutical compositions to restore the physiological processes in an organism, and also rejuvenation and prolongation of life of the tested animal.

The literary base of C.elegans correspondence as a model for researches.

Nematode Caenorhabditis elegans is multicellular genetic model for researching fundamental questions in biology. Its reproductive cycle is relatively short and an animal which may be easily kept in laboratory conditions. As a human organism, C. elegans grows, develops (from mature single-cell oocyte into multicellular adult organism), breeds, has a sensation, moves, learns, has a behaviour, grows old, falls ill, dies etc. - i.e. it shows the basic biological traits which characterize the highest living systems. Its genetic material (DNA) and duration of cells development are well-known, that allows to study with effect functioning of genes and proteins at the level of a single cell. About 50% of genes C. elegans have human homology. Functional analysis of these genes resulted in discovery of several evolutionary significant biological lows, for example, genetics of apoptosis (programmed death of cells) and differentiation of the cell destiny by means of the cellular signals. These important discoveries were awarded with Nobel prize in 2002 and 2006. Our purpose is to present C. elegans in industrial, clinical and biological research application.

### C. elegans as a genetic system

C. elegans is a microscopic organism with 1.2 mm length. The term of its reproduction is relatively short (3 days at 25°C) and, as all the microorganisms, it may be kept in great amount on a plastic plate. The kinds of nematode may be also kept during indefinite time in liquid nitrogen. C. elegans exists in two sexual forms: self-matured hermaphrodites are a source of pure genetic colonies, at that the males make possible mating between different genetic colonies. An animal consists of about 1.000 somatic cells, with the term of development (cellular age) is invariative and totally determined. 3-dimensional anatomical structure of the nervous system of a worm (contains 302 neurons) and a network of its nerves are well-known. Since under the light microscope a body of C. elegans is transparent, at the level of single cell it is possible to easily determine both normal and pathological events in development.

Genome of C. elegans is decoded and counts about 20.000 genes, the functional analysis of which already contains very detailed data. 8.000 genes of a worm are for the present characterized concerning mutation, but the attempts to generate perfectly functioning form of potentially every gene continue (Gene Knockout Project). Simultaneously, the scientific method on the base of the sequence of genes allowed to inactivate a code potential of primary genome of a worm (Gene Knockout Project). Now the program of protein-protein interaction and a map of systematic expression of genes are revealed. A vast potential of combination of genetics, cellular biology and molecular biology made C. elegans an attractive system model in researching biology. Thanks to presence of a great amount of genic conformity between a worm and a human system C. Elegans became one of the most popular models of examination of serious human diseases. (See Olsen et al Caenorhabditis elegans as a Model for Aging and Age-Related Diseases published in Annals of the New York Academy of Sciences and Huang et al PNAS May 25, also Cheng et al PNAS 2004 vol. 101 no. 21 8084-8089 Measurements of the Age-related Changes of Physiological Processes that Predict Lifespan of C. elegans).

### Materials and methods:

In the researches the compositions containing SBECD and ADAM QD suspension of SiO₂ and TiO₂ activated particles were used.
1. ADAM QD/T, TiO₂ aqueous nanodispersion used for researches on the animals, was sterile nanodispersion, of pharmaceutical class, in concentration 0.6 mg/ml.
2. ADAM QD/S, SiO₂ aqueous nanodispersion used for researches on the animals, was sterile solution, in concentration 0.6 mg/ml.
3. SBECD, Sulfobutylated beta-cyclodextrin sodium salt, Budapest, Hungary DEXOLVE ^{®} pharma grade (DS~6.5), powder in concentration 0.3 mg/ml.

### Protocol of researches and step-by-step methodology:

- embryos were isolated (synchronization)
- larvae of identical phase (L4) were placed on the standard NGM (nematode growth medium) agarinic plates in the Petri dishes.
- all the animals were in the age from first up to ninth day of life and lived in a medium containing the above-listed components, after ninth day they were placed in standard NGM agarinic plate without other additions as FUDR.
- deaths of the animals were constantly registered as a time function
- the curves of life duration were created on the basis of read indications.

Composition of NGM plates (all the chemical compounds and reagents of analytical class); NaCl, agar, peptone, 5 mg/ml cholesterol in ethanol, non-autoclaved, 1 M K₃PO₄ buffer pH 6.0 (108.3 g KH₂PO₄, 35.6 g K₂HPO₄, H₂O for 1 liter), 1M MgSO₄, Petri dishes, peristaltic pump.

### Conclusions:

The results present average of three parallel, independent definitions, research cycles of C. elegans.

The effect of ADAM QD/T, TiO₂ on life duration of C. elegans:
Heterocrystalline suspension TiO₂ in concentration 0.6 mg/ml, used in a standard cultural medium (NGM), showed significant improvement of life duration and prolongation of youth during a term of life.

The animals treated by TiO₂ suspension remained young and, correspondingly, kept "life quality" for a longer time.

Analogous tendencies were marked on a wild kind of C elegans, treated by SiO₂ suspension in analogous conditions.

Combination of TiO₂, SiO₂ nanodispersions, with concentration 0.6 mg/ml with SBECD 3 mg/ml still more enlarged life duration and breeding of animals.

Besides registration of a number of animals, survival factor of C. elegans was determined as a time function, moreover, examination of C. elegans gave possibility to reveal the process of aging with a help of fluorescent microscopic research of animals histology. Since the process of aging always correlates with forming fluorescent senile pigment (generally, lipofuscin) in interstitial tissues of C.elegans, use of appropriate microscopy immediately gives a real picture of animals life in the course of their aging.

In the result of executed microscopy, using the light microscope Nomarski, C. elegans fluorescent image of a young adult animal, in 3 days age, does not show senile fluorescence in interstitial tissues.

In the result of executed microscopy, using the light microscope Nomarski, fluorescent image of a young adult animal, in 14 days age, C. elegans shows self-luminous senile pigment in interstitial tissues (often lipofuscin accumulation).

As the Table 16 shows, a positive effect of treatment by suspensions ADAM QD/T TiO₂, ADAM QD/S, SiO₂ and SBECD 3 mg/ml on life duration of C. elegans was noted. In the result of administration of TiO₂ in the medium to C. elegans, life duration of animals enlarged by 20% in comparison with a control group, and by 23% using TiO₂+ SBECD.

Combined administration of polydisperse suspensions, taken in amount 0.6 mg/ml, and SBECD taken in amount 0.3 mg/ml, into the matrix of a cultural medium NGM showed extension of life duration of an animal, and also rejuvenating effect on wild kind of C. Elegans.

**Table 16**

| Life duration (days) | Number of living C.elegans (%) C. elegans | | | | | |
|---|---|---|---|---|---|---|
| | Control of NGM | TiO₂ | SiO₂ | SBECD | TiO₂ SBECD | SiO₂ SBECD |
| 10 | 90 | 96 | 94 | 90 | 100 | 98 |
| 13 | 42 | 44 | 40 | 41 | 45 | 42 |
| 14 | 20 | 28 | 21 | 20 | 31 | 22 |
| 15 | 8 | 17 | 9 | 7 | 22 | 21 |
| 18 | 0 | 20 | 2 | 0 | 23 | 20 |

From the experiments results of the present research it follows that:
- Examinations of life duration of C. elegans were executed in appropriate way and on the base of a reliable method of researches for detection of rejuvenating effect of nanoparticles of aqueous suspension of TiO2, SiO2 and its combinations with SBECD.
- Pharmaceutical combinations taken in said concentrations in cultural medium NGM showed positive effect on life duration of different (mutants and wild kinds) types of C. elegans. Rendering rejuvenating effect in oral use, may have practically useful rejuvenating qualities.
- This observation gives confirmation in additional prolonged injection researches of biological organisms, that efficiency on the base of fluorescent evaluation of senile pigments, for example, spectrophotometric tracing of lipofuscin accumulation in the course of treatment, is considerably higher that in oral use.

## Claims

1. Pharmaceutical composition for restoring the physiological processes and the cells of an organism, **characterized by** presence of cyclodextrins - cyclic maltooligosaccharides chosen from, alpha-cyclodextrins, beta-cyclodextrins, gamma-cyclodextrins, randomly methylated beta-cyclodextrins, 2-hydroxypropyl-beta-cyclodextrins, sulfobutylated beta-cyclodextrins, 2-hydroxypropyl-gamma-cyclodextrins or their mixtures and sugammadex, used in amount 0.001-99.9 mass% in combination with aqueous stable suspension of heterocrystals of titanium dioxide or silicon dioxide particles with size not less than 450 nm and content in amount of 0.0001 - 5% mass, having on the surface of crystals and particles in the energy centers electronically-excited triplet oxygen ³O₂.

2. Pharmaceutical composition according to claim 1, **characterized in that** the composition additionally comprises active pharmaceutical ingredients and pharmaceutically acceptable additives in amount 0.0001 - 99.0% mass.

3. Pharmaceutical composition according to the claim 1, **characterized in** presence on the lattice surface of titanium dioxide and silicon dioxide electronically-excited triplet oxygen ³O₂ in the energy centers, namely, in the quantum dots - zones of local overheating, ensuring catalytic activity for formation in an organism of reactive species of oxygen .

4. Pharmaceutical composition according to claims 1 and 2, **characterized in** additional content of cytostatic Doxorubicin taken in amount from 0.0001 up to 0.001 mg/ml for intramuscular administering.

5. Pharmaceutical composition according to claims 1 and 2, **characterized in that** in adjuvant therapy pharmaceutical composition is used separately or active pharmaceutical ingredients and pharmaceutically acceptable additives.

6. Pharmaceutical composition according to claims 1 and 2, **characterized in** administration - intravenously, intramuscularly, perorally, nasally, vaginally, rectally, ENT- or local administration, and use in the form of powders, tableted forms, ointments, creams, lotions, solutions, plasters and aerosols.

7. Pharmaceutical composition according to claims 1 and 2, **characterized in** use, in particular, in the form of medical products of topical use in the form of hydrocolloidal medical plasters, impregnation for tampons, absorbent pads, diapers, tapes or bandages.

8. Medical agent, comprising pharmaceutical composition according to claims 1, 2 and 5, **characterized in** ensuring therapeutically significant effect for restoring physiological processes and cells of organism.

## Patentansprüche

1. Pharmazeutische Zusammensetzung zum Wiederherstellen der physiologischen Prozesse und der Zellen eines Organismus, **gekennzeichnet durch** das Vorhandensein von Cyclodextrinen - zyklischen Maltooligosacchariden, ausgewählt aus Alpha-Cyclodextrinen, Beta-Cyclodextrinen, Gamma-Cyclodextrinen, zufällig methylierten Beta-Cyclodextrinen, 2-Hydroxypropyl-Beta-Cyclodextrinen, sulfobutylierten Beta-Cyclodextrinen, 2-Hydroxypropyl-Gamma-Cyclodextrinen oder deren Mischungen und Sugammadex, verwendet in einer Menge von 0.001 - 99.9 Masse% in Kombination mit einer wässrigen stabilen Suspension von Heterokristallen von Titandioxid- oder Siliziumdioxidpartikeln mit einer Größe von nicht kleiner als 450 nm und einem Gehalt in einer Menge von 0.0001 - 5% Masse, mit elektronisch angeregtem Triplett-Sauerstoff ³O₂ auf der Oberfläche der Kristalle und Partikel in den Energiezentren.

2. Pharmazeutische Zusammensetzung gemäß Anspruch 1, **dadurch gekennzeichnet, dass** die Zusammensetzung zusätzlich pharmazeutische Inhaltsstoffe und pharmazeutisch akzeptable Zusatzstoffe in einer Menge von 0.0001 - 99.0% Masse umfasst.

3. Pharmazeutische Zusammensetzung gemäß Anspruch 1, **gekennzeichnet durch** das Vorhandensein von Titandioxid und Siliziumdioxid auf der Gitteroberfläche des elektronisch angeregten Triplett-Sauerstoff ³O₂ in den Energiezentren, nämlich in den Quantenpunkten - Zonen von lokaler Überhitzung, die eine katalytische Aktivität für die Bildung von reaktiven Sauerstoffspezies in einem Organismus gewährleisten.

4. Pharmazeutische Zusammensetzung gemäß den Ansprüchen 1 und 2, **gekennzeichnet durch** einen zusätzlichen Gehalt an zytostatischem Doxorubicin, das in einer Menge von 0.0001 bis 0.001 mg/ml für das intramuskuläre Verabreichen verwendet wird.

5. Pharmazeutische Zusammensetzung gemäß den Ansprüchen 1 und 2, **dadurch gekennzeichnet, dass** in der adjuvanten Therapie die pharmazeutische Zusammensetzung getrennt oder aktive pharmazeutische Inhaltsstoffe und pharmazeutisch akzeptable Zusatzstoffe verwendet werden.

6. Pharmazeutische Zusammensetzung gemäß den Ansprüchen 1 und 2, **gekennzeichnet durch** Verabreichung - intravenös, intramuskulär, peroral, nasal, vaginal, rektal, HNO- oder lokale Verabreichung, und Verwendung in der Form von Pulvern, Tablettenformen, Salben, Cremes, Lotionen, Lösungen, Pflastern und Aerosolen.

7. Pharmazeutische Zusammensetzung gemäß den Ansprüchen 1 und 2, **gekennzeichnet durch** die Verwendung insbesondere in der Form von medizinischen Produkten zur äußerlichen Anwendung in der Form von hydrokolloidalen medizinischen Pflastern, Imprägnierungen für Tampons, absorbierenden Binden, Windeln, Bänder oder Bandagen.

8. Medizinisches Mittel, das eine pharmazeutische Zusammensetzung gemäß den Ansprüchen 1, 2 und 5 umfasst, **gekennzeichnet durch** das Gewährleisten einer therapeutisch signifikanten Wirkung zum Wiederherstellen physiologischer Prozesse und Zellen des Organismus

## Revendications

1. Composition pharmaceutique destinée à restaurer les processus physiologiques et les cellules d'un organisme, **caractérisée par** la présence de cyclodextrines - des maltooligosaccharides cycliques choisis parmi des alpha-cyclodextrines, des bêta-cyclodextrines, des gamma-cyclodextrines, des bêta-cyclodextrines méthylées aléatoirement, des 2- hydroxypropyl-bêta-cyclodextrines, des bêta-cyclodextrines sulfobutylées, des 2-hydroxypropyl-gamma-cyclodextrines ou leurs mélanges et sugammadex, utilisées en une quantité de 0,001 à 99,9 % en masse en combinaison avec une suspension stable aqueuse d'hétérocristaux de particules de dioxyde de titane ou de dioxyde de silicium ayant une taille non inférieure à 450 nm et une teneur en une quantité de 0,0001 à 5 % en masse, ayant sur la surface des cristaux et des particules dans les centres énergétiques de l'oxygène triplet excité électroniquement ³O₂.

2. Composition pharmaceutique selon la revendication 1, **caractérisée en ce que** la composition comporte en plus des ingrédients pharmaceutiques actifs et des additifs acceptables du point de vue pharmaceutique en une quantité de 0,0001 à 99,0 % en masse.

3. Composition pharmaceutique selon la revendication 1, **caractérisée par** la présence sur la surface réticulaire du dioxyde de titane et du dioxyde de silicium d'oxygène triplet excité électroniquement ³O₂ dans les centres énergétiques, à savoir, dans les points quantiques - des zones de surchauffe locale, assurant une activité catalytique pour la formation dans un organisme d'espèces réactives de l'oxygène.

4. Composition pharmaceutique selon les revendications 1 et 2, **caractérisée par** une teneur additionnelle en doxorubicine cytostatique prise en une quantité de 0,0001 jusqu'à 0,001 mg/ml pour une administration intramusculaire.

5. Composition pharmaceutique selon les revendications 1 et 2, **caractérisée en ce qu'**en traitement d'appoint, une composition pharmaceutique est utilisée séparément ou des ingrédients pharmaceutiques actifs et des additifs acceptables du point de vue pharmaceutique.

6. Composition pharmaceutique selon les revendications 1 et 2, **caractérisée par** une administration - de manière intraveineuse, intramusculaire, pérorale, nasale, vaginale, rectale, ENT - ou une administration locale, et une utilisation sous la forme de poudres, de comprimés, d'onguents, de crèmes, de lotions, de solutions, de plâtres et d'aérosols.

7. Composition pharmaceutique selon les revendications 1 et 2, **caractérisée par** une utilisation, en particulier sous la forme de produits médicaux à usage topique sous la forme de plâtres médicaux hydrocolloïdes, d'imprégnation pour tampons, tampons absorbants, couches, bandes ou bandages.

8. Agent médical, comportant une composition pharmaceutique selon les revendications 1, 2 et 5, **caractérisé en ce qu'**il assure un effet thérapeutiquement significatif pour restaurer des processus physiologiques et des cellules d'organisme.
